# EUROPEAN PATENT SPECIFICATION

(11) **EP 1 799 248 B1**
(45) Date of publication and mention of the grant of the patent: **08.04.2009**
(21) Application number: 05786995.0
(22) Date of filing: 21.09.2005
(51) Int. Cl.: A61K 38/20, A61K 38/21, A61P 25/00

(54) **USE OF IL-17F FOR THE TREATMENT AND/OR PREVENTION OF NEUROLOGIC DISEASES**
VERWENDUNG VON IL-17F ZUR BEHANDLUNG UND/ODER PRÄVENTION VON NEUROLOGISCHEN ERKRANKUNGEN
UTILISATION DU IL-17F DANS LE TRAITEMENT ET/OU LA PRÉVENTION DE MALADIES NEUROLOGIQUES

(30) Priority: 21.09.2004 EP 04104573; 22.11.2004 US 630105 P
(43) Date of publication of application: 27.06.2007
(73) Proprietor: Merck Serono SA, 1267 Coinsins, Vaud (CH)
(72) Inventor: SAGOT, Yves, F-74160 Beaumont (FR); POULY, Sandrine, CH-1207 Genève (CH)
(74) Representative: Merck Serono SA - Geneva Intellectual Property
(86) International application number: PCT/EP2005/054719
(87) International publication number: WO 2006/032673

(56) References cited:
- WO-A-01/46420
- US-A1- 2002 182 673
- US-A1- 2003 215 388
- KAWAGUCHI MIO ET AL: "Structural and functional analysis of a new cytokine, ML-1 (interleukin-17F)." ALLERGOLOGY INTERNATIONAL, vol. 52, no. 3, September 2003 (2003-09), pages 117-122, XP001206022 ISSN: 1323-8930
- DATABASE BIOSIS [Online] BIOSCIENCES INFORMATION SERVICE, PHILADELPHIA, PA, US; 1 October 2001 (2001-10-01), HYMOWITZ SARAH G ET AL: "IL-17s adopt a cystine knot fold: Structure and activity of a novel cytokine, IL-17F, and implications for receptor binding" XP001206020 Database accession no. PREV200100514235 & EMBO (EUROPEAN MOLECULAR BIOLOGY ORGANIZATION) JOURNAL, vol. 20, no. 19, 1 October 2001 (2001-10-01), pages 5332-5341, ISSN: 0261-4189
- EBERL M: "Don't count your interleukins before they've hatched" TRENDS IN IMMUNOLOGY, ELSEVIER, CAMBRIDGE, GB, vol. 23, no. 7, 1 July 2002 (2002-07-01), pages 341-342, XP004367749 ISSN: 1471-4906
- STARNES T ET AL: "Cutting edge: IL-17F, a novel cytokine selectively expressed in activated T cells and monocytes, regulates angiogenesis and endothelial cell cytokine production." JOURNAL OF IMMUNOLOGY (BALTIMORE, MD. : 1950) 15 OCT 2001, vol. 167, no. 8, 15 October 2001 (2001-10-15), pages 4137-4140, XP001206023 ISSN: 0022-1767
- KAWAGUCHI M ET AL: "IL-17 cytokine family" JOURNAL OF ALLERGY AND CLINICAL IMMUNOLOGY, MOSBY - YEARLY BOOK, INC, US, vol. 114, no. 6, December 2004 (2004-12), pages 1265-1273, XP004666361 ISSN: 0091-6749

### SEQUENCE LISTING

<110> Applied Research Systems ARS Holding N.V.
<120> USE OF IL-17F FOR THE TREATMENT AND/OR PREVENTION OF NEUROLOGIC DISEASES
<130> 1004 WO
<150> EP04104573.3
   <151> 2004-09-21
<160> 2
<170> PatentIn version 3.3
<210> 1
   <211> 163
   <212> PRT
   <213> Homo sapiens
<400> 1
   Met Thr Val Lys Thr Leu His Gly Pro Ala Met Val Lys Tyr Leu Leu
   1               5                   10                  15
   Leu Ser Ile Leu Gly Leu Ala Phe Leu Ser Glu Ala Ala Ala Arg Lys
               20                  25                  30
   Ile Pro Lys Val Gly His Thr Phe Phe Gln Lys Pro Glu Ser Cys Pro
           35                  40                  45
   Pro Val Pro Gly Gly Ser Met Lys Leu Asp Ile Gly Ile Ile Asn Glu
       50                  55                  60
   Asn Gln Arg Val Ser Met Ser Arg Asn Ile Glu Ser Arg Ser Thr Ser
   65                  70                  75                  80
   Pro Trp Asn Tyr Thr Val Thr Trp Asp Pro Asn Arg Tyr Pro Ser Glu
                   85                  90                  95
   Val Val Gln Ala Gln Cys Arg Asn Leu Gly Cys Ile Asn Ala Gln Gly
               100                 105                 110
   Lys Glu Asp Ile Ser Met Asn Ser Val Pro Ile Gln Gln Glu Thr Leu
           115                 120                 125
   Val Val Arg Arg Lys His Gln Gly Cys Ser Val Ser Phe Gln Leu Glu
       130                 135                 140
   Lys Val Leu Val Thr Val Gly Cys Thr Cys Val Thr Pro Val Ile His
   145                 150                 155                 160
   His Val Gln
<210> 2
   <211> 492
   <212> DNA
   <213> Homo sapiens
<400> 2
   atgacagtga agaccctgca tggcccagcc atggtcaagt acttgctgct gtcgatattg     60
   gggcttgcct ttctgagtga ggcggcagct cggaaaatcc ccaaagtagg acatactttt    120
   ttccaaaagc ctgagagttg cccgcctgtg ccaggaggta gtatgaagct tgacattggc    180
   atcatcaatg aaaaccagcg cgtttccatg tcacgtaaca tcgagagccg ctccacctcc    240
   ccctggaatt acactgtcac ttgggacccc aaccggtacc cctcggaagt tgtacaggcc    300
   cagtgtagga acttgggctg catcaatgct caaggaaagg aagacatctc catgaattcc    360
   gttcccatcc agcaagagac cctggtcgtc cggaggaagc accaaggctg ctctgtttct    420
   ttccagttgg agaaggtgct ggtgactgtt ggctgcacct gcgtcacccc tgtcatccac    480
   catgtgcagt aa                                                        492

## Description

### FIELD OF THE INVENTION

The present invention is generally in the field of neurologic diseases and disorders. It relates to neuroprotection, nerve myelination and generation or re-generation of myelin producing cells. In particular, it relates to demyelinating and neurodegenerative diseases, neuropathies, traumatic nerve injury, stroke and neurologic diseases caused by congenital metabolic disorders. More specifically, the present invention relates to the use of IL-17F, for the manufacture of a medicament for treatment and/or prevention of a neurologic disease.

### BACKGROUND OF THE INVENTION

Nerve myelination is an essential process in the formation and function of the central nervous system (CNS) and peripheral nervous system (PNS) compartments. The myelin sheath around axons is necessary for the proper conduction of electric impulses along nerves. Loss of myelin occurs in a number of diseases, among which are Multiple Sclerosis (MS) affecting the CNS, Guillain-Barre Syndrome, Chronic Immune Demyelinating Polyneuropathy (CIDP) and others (see Abramsky and Ovadia, 1997; Hartung et al., 1998). While of various etiologies, such as infectious pathogens or autoimmune attacks, demyelinating diseases all cause loss of neurologic function and may lead to paralysis and death. While present therapeutical agents reduce inflammatory attacks in MS and retard disease progression, there is a need to develop therapies that could lead to remyelination and recovery of neurologic function (Abramsky and Ovadia, 1997).

Injury to CNS induced by acute insults including trauma, hypoxia and ischemia can affect both neurons and white matter. Although most attention has been paid to processes leading to neuronal death, increasing evidence suggests that damage to oligodendrocytes, which myelinate axons, is also a specific component of CNS injury. Thus oligodendrocyte pathology was demonstrated at very early phase after stroke (3 hours) in rats, suggesting that these cells are even more vulnerable to excitotoxic events than neuronal cells (Pantoni et al., 1996). One potential candidate mediating cell death is the marked elevation of glutamate concentration that accompanies many acute CNS injuries (Lipton et al. 1994). Indeed, beside neurons oligodendrocytes were also found to express functional glutamate receptors belonging to the AMPA/kainate subtype. Moreover oligodendrocytes display high vulnerability to glutamate application (McDonald et al., 1998).

Trauma is an injury or damage of the nerve. It may be spinal cord trauma, which is damage to the spinal cord that affects all nervous functions that are controlled at and below the level of the injury, including muscle control and sensation, or brain trauma, such as trauma caused by closed head injury.

Cerebral hypoxia is a lack of oxygen specifically to the cerebral hemispheres, and more typically the term is used to refer to a lack of oxygen to the entire brain. Depending on the severity of the hypoxia, symptoms may range from confusion to irreversible brain damage, coma and death.

Stroke is usually caused by ischemia of the brain. It is also called cerebrovascular disease or accident. It is a group of brain disorders involving loss of brain functions that occurs when the blood supply to any part of the brain is interrupted. The brain requires about 20% of the circulation of blood in the body. The primary blood supply to the brain is through 2 arteries in the neck (the carotid arteries), which then branch off within the brain to multiple arteries that each supply a specific area of the brain. Even a brief interruption to the blood flow can cause decreases in brain function (neurologic deficit). The symptoms vary with the area of the brain affected and commonly include such problems as changes in vision, speech changes, decreased movement or sensation in a part of the body, or changes in the level of consciousness. If the blood flow is decreased for longer than a few seconds, brain cells in the area are destroyed (infarcted) causing permanent damage to that area of the brain or even death.

A stroke affects about 4 out of 1,000 people. It is the 3rd leading cause of death in most developed countries, including the U.S. The incidence of stroke rises dramatically with age, with the risk doubling with each decade after age 35. About 5% of people over age 65 have had at least one stroke. The disorder occurs in men more often than women.

As mentioned above, a stroke involves loss of brain functions (neurologic deficits) caused by a loss of blood circulation to areas of the brain. The specific neurologic deficits may vary depending on the location, extent of the damage, and cause of the disorder. A stroke may be caused by reduced blood flow (ischemia) that results in deficient blood supply and death of tissues in that area (infarction). Causes of ischemic strokes are blood clots that form in the brain (thrombus) and blood clots or pieces of atherosclerotic plaque or other material that travel to the brain from another location (emboli). Bleeding (hemorrhage) within the brain may cause symptoms that mimic stroke.

The most common cause of a stroke is stroke secondary to atherosclerosis (cerebral thrombosis). Atherosclerosis ("hardening of the arteries") is a condition in which fatty deposits occur on the inner lining of the arteries, and atherosclerotic plaque (a mass consisting of fatty deposits and blood platelets) develops. The occlusion of the artery develops slowly. Atherosclerotic plaque does not necessarily cause a stroke. There are many small connections between the various brain arteries. If the blood flow gradually decreases, these small connections will increase in size and "by-pass" the obstructed area (collateral circulation). If there is enough collateral circulation, even a totally blocked artery may not cause neurologic deficits. A second safety mechanism within the brain is that the arteries are large enough that 75% of the blood vessel can be occluded, and there will still be adequate blood flow to that area of the brain.

A thrombotic stroke (stroke caused by thrombosis) is most common in elderly people, and often there is underlying atherosclerotic heart disease or diabetes mellitus. This type of stroke may occur at any time, including at rest. The person may or may not lose consciousness.

Strokes caused by embolism (moving blood clot) are most commonly strokes secondary to a cardiogenic embolism, clots that develop because of heart disorders that then travel to the brain. An embolism may also originate in other areas, especially where there is atherosclerotic plaque. The embolus travels through the bloodstream and becomes stuck in a small artery in the brain. This stroke occurs suddenly with immediate maximum neurologic deficit. It is not associated with activity levels and can occur at any time. Arrhythmias of the heart are commonly seen with this disorder and often are the cause of the embolus. Damage to the brain is often more severe than with a stroke caused by cerebral thrombosis. Consciousness may or may not be lost. The probable outcome is worsened if blood vessels damaged by stroke rupture and bleed (hemorrhagic stroke).

Peripheral Neuropathy is a syndrome of sensory loss, muscle weakness and atrophy, decreased deep tendon reflexes, and vasomotor symptoms, alone or in any combination.

The disease may affect a single nerve (mononeuropathy), two or more nerves in separate areas (multiple mononeuropathy), or many nerves simultaneously (polyneuropathy). The axon may be primarily affected (e.g. in diabetes mellitus, Lyme disease, uremia or with toxic agents) or the myelin sheath or Schwann cell (e.g. in acute or chronic inflammatory polyneuropathy, leukodystrophies, or Guillain-Barré syndrome). Damage to small unmyelinated and myelinated fibers results primarily in loss of temperature and pain sensation; damage to large myelinated fibers results in motor or proprioceptive defects. Some neuropathies (e.g. due to lead toxicity, dapsone use, Lyme disease (caused by tick bite), porphyria, or Guillain-Barré syndrome) primarily affect motor fibers; others (e.g. due to dorsal root ganglionitis of cancer, leprosy, AIDS, diabetes mellitus, or chronic pyridoxine intoxication) primarily affect the dorsal root ganglia or sensory fibers, producing sensory symptoms. Occasionally, cranial nerves are also involved (e.g. in Guillain-Barré syndrome, Lyme disease, diabetes mellitus, and diphtheria). Identifying the modalities involved helps determine the cause.

Trauma is the most common cause of a localized injury to a single nerve. Violent muscular activity or forcible overextension of a joint may produce a focal neuropathy, as may repeated small traumas (e.g. tight gripping of small tools, excessive vibration from air hammers). Pressure or entrapment paralysis usually affects superficial nerves (ulnar, radial, peroneal) at bony prominences (e.g. during sound sleep or during anesthesia in thin or cachectic persons and often in alcoholics) or at narrow canals (e.g. in carpal tunnel syndrome). Pressure paralysis may also result from tumors, bony hyperostosis, casts, crutches, or prolonged cramped postures (e.g. in gardening). Hemorrhage into a nerve and exposure to cold or radiation may cause neuropathy. Mononeuropathy may result from direct tumor invasion.

Multiple mononeuropathy is usually secondary to collagen vascular disorders (e.g. polyarteritis nodosa, SLE, Sjögren's syndrome, RA), sarcoidosis, metabolic diseases (e.g. diabetes, amyloidosis), or infectious diseases (e.g. Lyme disease, HIV infection). Microorganism may cause multiple mononeuropathy by direct invasion of the nerve (e.g. in leprosy).

Polyneuropathy due to acute febrile diseases may result from a toxin (e.g. in diphtheria) or an autoimmune reaction (e.g. in Guillain-Barré syndrome); the polyneuropathy that sometimes follows immunizations is probably also autoimmune.

Toxic agents generally cause polyneuropathy but sometimes mononeuropathy. They include emetine, hexobarbital, barbital, chlorobutanol, sulfonamides, phenytoin, nitrofurantoin, the vinca alkaloids, heavy metals, carbon monoxide, triorthocresyl phosphate, orthodinitrophenol, many solvents, other industrial poisons, and certain AIDS drugs (e.g. zalcitabine, didanosine).

Chemotherapy-induced neuropathy is a prominent and serious side effect of several commonly used chemotherapy medications, including the Vinca alkaloids (vinblastine, vincristine and vindesine), platinum- containing drugs (cisplatin) and Taxanes (paclitaxel). The induction of peripheral neuropathy is a common factor in limiting therapy with chemotherapeutic drugs.

Nutritional deficiencies and metabolic disorders may result in polyneuropathy. B vitamin deficiency is often the cause (e.g. in alcoholism, beriberi, pernicious anemia, isoniazid-induced pyridoxine deficiency, malabsorption syndromes, and hyperemesis gravidarum). Polyneuropathy also occurs in hypothyroidism, porphyria, sarcoidosis, amyloidosis, and uremia. Diabetes mellitus can cause sensorimotor distal polyneuropathy (most common), multiple mononeuropathy, and focal mononeuropathy (e.g. of the oculomotor or abducens cranial nerves).

Malignancy may cause polyneuropathy via monoclonal gammopathy (multiple myeloma, lymphoma), amyloid invasion, or nutritional deficiencies or as a paraneoplastic syndrome.

Specific mononeuropathies: Single and multiple mononeuropathies are characterized by pain, weakness, and paresthesias in the distribution of the affected nerve. Multiple mononeuropathy is asymmetric; the nerves may be involved all at once or progressively. Extensive involvement of many nerves may simulate a polyneuropathy.

Ulnar nerve palsy is often caused by trauma to the nerve in the ulnar groove of the elbow by repeated leaning on the elbow or by asymmetric bone growth after a childhood fracture (tardy ulnar palsy). The ulnar nerve can also be compressed at the cubital tunnel. Paresthesias and a sensory deficit in the 5th and medial half of the 4th fingers occur; the thumb adductor, 5th finger abductor, and interossei muscles are weak and atrophied. Severe chronic ulnar palsy produces a clawhand deformity. Nerve conduction studies can identify the site of the lesion. Conservative treatment should be attempted before surgical repair is attempted.

The carpal tunnel syndrome results from compression of the median nerve in the volar aspect of the wrist between the transverse superficial carpal ligament and the longitudinal tendons of forearm muscles that flex the hand. It may be unilateral or bilateral. The compression produces paresthesias in the radial-palmar aspect of the hand and pain in the wrist and palm; sometimes pain occurs proximally to the compression site in the forearm and shoulder. Pain may be more severe at night. A sensory deficit in the palmar aspect of the first three fingers may follow; the muscles that control thumb abduction and opposition may become weak and atrophied. This syndrome should be distinguished from C-6 root compression due to cervical radiculopathy.

Peroneal nerve palsy is usually caused by compression of the nerve against the lateral aspect of the fibular neck. It is most common in emaciated bedridden patients and in thin persons who habitually cross their legs. Weakness of foot dorsiflexion and eversion (footdrop) occurs. Occasionally, a sensory deficit occurs over the anterolateral aspect of the lower leg and dorsum of the foot or in the web space between the 1st and 2nd metatarsals. Treatment is usually conservative for compressive neuropathies (e.g. avoiding leg crossing). Incomplete neuropathies are usually followed clinically and usually improve spontaneously. If recovery does not occur, surgical exploration may be indicated.

Radial nerve palsy (Saturday night palsy) is caused by compression of the nerve against the humerus, e.g. as the arm is draped over the back of a chair during intoxication or deep sleep. Symptoms include weakness of wrist and finger extensors (wristdrop) and, occasionally, sensory loss over the dorsal aspect of the 1st dorsal interosseous muscle. Treatment is similar to that of compressive peroneal neuropathy.

Polyneuropathies are relatively symmetric, often affecting sensory, motor, and vasomotor fibers simultaneously. They may affect the axon cylinder or the myelin sheath and, in either form, may be acute (e.g. Guillain-Barré syndrome) or chronic (e.g. renal failure).

Polyneuropathy due to metabolic disorders (e.g. diabetes mellitus) or renal failure develops slowly, often over months or years. It frequently begins with sensory abnormalities in the lower extremities that are often more severe distally than proximally. Peripheral tingling, numbness, burning pain, or deficiencies in joint proprioception and vibratory sensation are often prominent. Pain is often worse at night and may be aggravated by touching the affected area or by temperature changes. In severe cases, there are objective signs of sensory loss, typically with stocking-and-glove distribution. Achilles and other deep tendon reflexes are diminished or absent. Painless ulcers on the digits or Charcot's joints may develop when sensory loss is profound. Sensory or proprioceptive deficits may lead to gait abnormalities. Motor involvement results in distal muscle weakness and atrophy. The autonomic nervous system may be additionally or selectively involved, leading to nocturnal diarrhea, urinary and fecal incontinence, impotence, or postural hypotension. Vasomotor symptoms vary. The skin may be paler and drier than normal, sometimes with dusky discoloration; sweating may be excessive. Trophic changes (smooth and shiny skin, pitted or ridged nails, osteoporosis) are common in severe, prolonged cases.

Nutritional polyneuropathy is common among alcoholics and the malnourished. A primary axonopathy may lead to secondary demyelination and axonal destruction in the longest and largest nerves. Whether the cause is deficiency of thiamine or another vitamin (e.g. pyridoxine, pantothenic acid, folic acid) is unclear. Neuropathy due to pyridoxine deficiency usually occurs only in persons taking isoniazid for tuberculosis; infants who are deficient or dependent on pyridoxine may have convulsions. Wasting and symmetric weakness of the distal extremities is usually insidious but can progress rapidly, sometimes accompanied by sensory loss, paresthesias, and pain. Aching, cramping, coldness, burning, and numbness in the calves and feet may be worsened by touch. Multiple vitamins may be given when etiology is obscure, but they have no proven benefit.

Uncommonly, an exclusively sensory polyneuropathy begins with peripheral pains and paresthesias and progresses centrally to a loss of all forms of sensation. It occurs as a remote effect of carcinoma (especially bronchogenic), after excessive pyridoxine ingestion (> 0.5 g/day), and in amyloidosis, hypothyroidism, myeloma, and uremia. The pyridoxine-induced neuropathy resolves when pyridoxine is discontinued.

Hereditary neuropathies are classified as sensorimotor neuropathies or sensory neuropathies. Charcot-Marie-Tooth disease is the most common hereditary sensorimotor neuropathy. Less common sensorimotor neuropathies begin at birth and result in greater disability. In sensory neuropathies, which are rare, loss of distal pain and temperature sensation is more prominent than loss of vibratory and position sense. The main problem is pedal mutilation due to pain insensitivity, with frequent infections and osteomyelitis.

Hereditary motor and sensory neuropathy types I and II (Charcot-Marie-Tooth disease, peroneal muscular atrophy) is a relatively common, usually autosomal dominant disorder characterized by weakness and atrophy, primarily in peroneal and distal leg muscles. Patients may also have other degenerative diseases (e.g. Friedreich's ataxia) or a family history of them. Patients with type I present in middle childhood with footdrop and slowly progressive distal muscle atrophy, producing "stork legs." Intrinsic muscle wasting in the hands begins later. Vibration, pain, and temperature sensation decreases in a stocking-glove pattern. Deep tendon reflexes are absent. High pedal arches or hammer toes may be the only signs in less affected family members who carry the disease. Nerve conduction velocities are slow, and distal latencies prolonged. Segmental demyelination and remyelination occur. Enlarged peripheral nerves may be palpated. The disease progresses slowly and does not affect life span. Type II disease evolves more slowly, with weakness usually developing later in life. Patients have relatively normal nerve conduction velocities but low amplitude evoked potentials. Biopsies show wallerian degeneration.

Hereditary motor and sensory neuropathy type III (hypertrophic interstitial neuropathy, Dejerine-Sottas disease), a rare autosomal recessive disorder, begins in childhood with progressive weakness and sensory loss and absent deep tendon reflexes. Initially, it resembles Charcot-Marie-Tooth disease, but motor weakness progresses at a faster rate. Demyelination and remyelination occur, producing enlarged peripheral nerves and onion bulbs seen on nerve biopsy.

Neurodegenerative diseases comprise, among others, Alzheimer's disease, Parkinson's disease, Huntington's disease and Amyotrophic Lateral Sclerosis (ALS).

Alzheimer's disease is a disorder involving deterioration in mental functions resulting from changes in brain tissue. This includes shrinking of brain tissues, not caused by disorders of the blood vessels, primary degenerative dementia and diffuse brain atrophy. Alzheimer's disease is also called senile dementia/Alzheimer's type (SDAT). It is the most common cause of intellectual decline with aging. The incidence is approximately 9 out of 10,000 people. This disorder affects women slightly more often than men and occurs primarily in older individuals.

The cause is unknown. The neurochemical factors which may participate in generation of the disease include lack of the substances used by the nerve cells to transmit nerve impulses (neurotransmitters), including acetylcholine, somatostatin, substance P, and norepinephrine. Environmental factors include exposure to aluminum, manganese, and other substances. The infectious factors include prion (virus-like organisms) infections that affect the brain and spinal cord (central nervous system). In some families (representing 5 to 10% of cases) there is an inherited predisposition to development of the disorder, but this does not follow strict (Mendelian) patterns of inheritance. The diagnosis is usually made by ruling out other causes of dementia.

Researchers have found that in families that have multiple members with Alzheimer's, there is a particular gene variation that is common to all of those with the disease. The gene, which produces a substance called apolipoprotein E4, is not said to cause the disease, its presence simply increases the chances that the disease may eventually occur. There are many people who have the E4 gene and never become afflicted with Alzheimer's.

The onset is characterized by impaired memory, with progressive loss of intellectual function. There may be mood changes, changes in language capability, changes in gait, and other changes as the disorder progresses. There is a decrease in the size (atrophy) of the tissues of the brain, enlargement of the ventricles (the spaces within the brain), and deposits within the tissues of the brain.

Parkinson's disease is a disorder of the brain characterized by shaking and difficulty with walking, movement, and coordination. The disease is associated with damage to a part of the brain that controls muscle movement. It is also called paralysis agitans or shaking palsy.

The disease affects approximately 2 out of 1,000 people, and most often develops after age 50. It affects both men and women and is one of the most common neurologic disorders of the elderly. The term "parkinsonism" refers to any condition that involves a combination of the types of changes in movement seen in Parkinson's disease, which happens to be the most common condition causing this group of symptoms. Parkinsonism may be caused by other disorders or by external factors (secondary parkinsonism).

Parkinson's disease is caused by progressive deterioration of the nerve cells of the part of the brain that controls muscle movement (the basal ganglia and the extrapyramidal area). Dopamine, which is one of the substances used by cells to transmit impulses (transmitters), is normally produced in this area. Deterioration of this area of the brain reduces the amount of dopamine available to the body. Insufficient dopamine disturbs the balance between dopamine and other transmitters, such as acetylcholine. Without dopamine, the nerve cells cannot properly transmit messages, and this results in the loss of muscle function. The exact reason that the cells of the brain deteriorate is unknown. The disorder may affect one or both sides of the body, with varying degrees of loss of function.

In addition to the loss of muscle control, some people with Parkinson's disease become severely depressed. Although early loss of mental capacities is uncommon, with severe Parkinson's the person may exhibit overall mental deterioration (including dementia, hallucinations, and so on). Dementia can also be a side effect of some of the medications used to treat the disorder.

Huntington's Disease (HD) is an inherited, autosomal dominant neurologic disease. The disease does not usually become clinically apparent until the fifth decade of life, and results in psychiatric disturbance, involuntary movement disorder, and cognitive decline associated with inexorable progression to death, typically 17 years following onset.

The gene responsible for Huntington's disease is called huntingtin. It is located on chromosome 4p, presenting an effective means of preclinical and antenatal diagnosis. The genetic abnormality consists in an excess number of tandemly repeated CAG nucleotide sequences. Other diseases with CAG repeats include, for example, spinal muscular atrophies (SMA), such as Kennedy's disease, and most of the autosomal dominant cerebellar ataxias (ADCAs) that are known as spinocerebellar ataxias (SCAs) in genetic nomenclature.

In HD, it is not known how this widely expressed gene, results in selective neuronal death. Further, sequence analysis revealed no obvious homology to other known genes and no structural motifs or functional domains were identified which clearly provide insights into its function. In particular, the question of how these widely expressed genes cause selective neuronal death remains unanswered.

Amyptrophic Lateral Sclerosis, ALS, is a disorder causing progressive loss of nervous control of voluntary muscles because of destruction of nerve cells in the brain and spinal cord. Amyotrophic Lateral Sclerosis, also called Lou Gehrig's disease, is a disorder involving loss of the use and control of muscles. The nerves controlling these muscles shrink and disappear, which results in loss of muscle tissue due to the lack of nervous stimulation. Muscle strength and coordination decreases, beginning with the voluntary muscles (those under conscious control, such as the muscles of the arms and legs). The extent of loss of muscle control continues to progress, and more and more muscle groups become involved. There may be a loss of nervous stimulation to semi-voluntary muscles, such as the muscles that control breathing and swallowing. There is no effect on ability to think or reason. The cause is unknown.

ALS affects approximately 1 out of 100,000 people. It appears in some cases to run in families. The disorder affects men more often than women. Symptoms usually do not develop until adulthood, often not until after age 50.

Traumatic nerve injury may concern the CNS or the PNS. Traumatic brain injury, also simply called head injury or closed head injury, refers to an injury where there is damage to the brain because of an external blow to the head. It mostly happens during car or bicycle accidents, but may also occur as the result of near drowning, heart attack, stroke and infections. This type of traumatic brain injury would usually result due to the lack of oxygen or blood supply to the brain, and therefore can be referred to as an "anoxic injury".

Brain injury or closed head injury occurs when there is a blow to the head as in a motor vehicle accident or a fall. In this case, the skull hits a stationary object and the brain turns and twists on its axis (the brain stem), causing localised or widespread damage. Also, the brain, a soft mass surrounded by fluid that allows it to "float," may rebound against the skull resulting in further damage.

There may be a period of unconsciousness immediately following the trauma, which may last minutes, weeks or months. Due to the twisting and rebounding, the traumatically brain injured patient usually receives damage or bruising to many parts of the brain. This is called diffuse damage, or "non-missile injury" to the brain. The types of brain damages occurring in non-missile injuries may be classified as either primary or secondary.

Primary brain damage occurs at the time of injury, mainly at the sites of impact, in particular when a skull fraction is present. Large contusions may be associated with an intracerebral haemorrhage, or accompanied by cortical lacerations. Diffuse axonal injuries occur as a result of shearing and tensile strains of neuronal processes produced by rotational movements of the brain within the skull. There may be small heamorrhagic lesions or diffuse damage to axons, which can only be detected microscopically.

Secondary brain damage occurs as a result of complications developing after the moment of injury. They include intracranial hemorrhage, traumatic damage to extracerebral arteries, intracranial herniation, hypoxic brain damage or meningitis.

An open head injury is a visible assault to the head and may result from a gunshot wound, an accident or an object going through the skull into the brain ("missile injury to the brain"), This type of head injury is more likely to damage a specific area of the brain.

So-called mild brain injury may occur with no loss of consciousness and possibly only a dazed feeling or confused state lasting a short time. Although medical care administered may be minimal, persons with brain injury without coma may experience symptoms and impairments similar to those suffered by the survivor of a coma injury.

In response to the trauma, changes occur in the brain, which require monitoring to prevent further damage. The brain's size frequently increases after a severe head injury. This is called brain swelling and occurs when there is an increase in the amount of blood to the brain. Later in the illness water may collect in the brain that is called brain edema. Both brain swelling and brain edema result in excessive pressure in the brain called intracranial pressure.

Spinal cord injuries account for the majority of hospital admissions for paraplegia and tetraplegia. Over 80% occur as a result of road accidents. Two main groups of injury are recognised clinially: open injuries and closed injuries.

Open injuries cause direct trauma of the spinal cord and nerve roots. Perforating injuries can cause extensive disruption and hemorrhage. Closed injuries account for most spinal injuries and are usually associated with a fracture/dislocation of the spinal column, which is usually demonstrable radiologically. Damage to the cord depends on the extent of the bony injuries and can be considered in two main stages: primary damage, which are contusions, nerve fibre transections and hemorrhagic necrosis, and secondary damage, which are extradural heamatoma, infarction, infection and edema.

Late effects of cord damage include: ascending and descending anterograde degeneration of damaged nerve fibers, post-traumatic syringomelyia, and systemic effects of paraplegia, such as urinary tract and chest infections, pressure sores and muscle wasting.

Neurologic disorders may further be due to congenital metabolic disorders. Myelin sheaths, which cover many nerve fibers, are composed of lipoprotein layers formed in early life. Myelin formed by the oligodendroglia in the CNS differs chemically and immunologically from that formed by the Schwann cells peripherally, but both types have the same function:
to promote transmission of a neural impulse along an axon.

Many congenital metabolic disorders (e.g. phenylketonuria and other aminoacidurias; Tay-Sachs, Niemann-Pick, and Gaucher's diseases; Hurler's syndrome; Krabbe's disease and other leukodystrophies) affect the developing myelin sheath, mainly in the CNS. Unless the biochemical defect can be corrected or compensated for, permanent, often widespread, neurologic deficits result.

For instrance, Krabbe's disease or globoid cell leukodystrophy is a disorder involving the white matter of the peripheral and central nervous systems. Mutations in the gene for the lysosomal enzyme galactocerebrosidase (GALC) result in low enzymatic activity and decreased ability to degrade galactolipids found almost exclusively in myelin.

Neurofibromatosis 1 (NF1) is a common autosomal disorder with a wide range of neurologic manifestations.

Multiple system atrophy (MSA) is a sporadic, adult-onset neurodegenerative disease of unknown etiology. The condition may be unique among neurodegenerative diseases by the prominent, if not primary, role played by the oligodendroglial cell in the pathogenetic process. The major difference to Parkinson's disease is that MSA patients do not respond to L-dopa treatment.

Demyelination in later life is a feature of many neurologic disorders; it can result from damage to nerves or myelin due to local injury, ischemia, toxic agents, or metabolic disorders. There is also evidence that demyelination may contribute to schizophrenia. Extensive myelin loss is usually followed by axonal degeneration and often by cell body degeneration, both of which may be irreversible. However, remyelination occurs in many instances, and repair, regeneration, and complete recovery of neural function can be rapid. Central demyelination (ie, of the spinal cord, brain, or optic nerves) is the predominant finding in the primary demyelinating diseases, whose etiology is unknown. The most well known is MS.

Acute disseminated encephalomyelitis, postinfectious encephalomyelitis is characterized by perivascular CNS demyelination, which can occur spontaneously but usually follows a viral infection or viral vaccination (or, very rarely, bacterial vaccination), suggesting an immunologic cause. Acute inflammatory peripheral neuropathies that follow a viral vaccination or the Guillain-Barré syndrome are similar demyelinating disorders with the same presumed immunopathogenesis, but they affect only peripheral structures.

Metachromatic leukodystrophy is another demyelinating disease. Adrenoleukodystrophy and adrenomyeloneuropathy are rare X-linked recessive metabolic disorders characterized by adrenal gland dysfunction and widespread demyelination of the nervous system. Adrenoleukodystrophy occurs in young boys; adrenomyeloneuropathy, in adolescents. Mental deterioration, spasticity, and blindness may occur. Adrenoleukodystrophy is invariably fatal. Dietary and immunomodulatory treatments are under study.

Leber's hereditary optic atrophy and related mitochondrial disorders are characterized primarily by bilateral loss of central vision, usually affecting young men in their late teens or early twenties. Leber's hereditary optic atrophy can resemble the optic neuritis in MS. Mutations in the maternally inherited mitochondrial DNA have been identified.

HTLV-associated myelopathy, a slowly progressive spinal cord disease associated with infection by the human T-cell lymphotrophic virus, is characterized by spastic weakness of both legs.

Further neurologic disorders comprise neuropathies with abnormal myelination, an overview of which is given below.

*Immune*: Acute, Guillain Barré, Chronic, Chronic Immune Demyelinating Polyneuropathy (CIDP), Multifocal CIDP, Multifocal Motor Neuropathy (MMN), Anti-MAG Syndrome, GALOP Syndrome, Anti-Sulfatide Antibody Syndrome (with serum M-protein), Anti-GM2 antibody syndrome, POEMS Syndrome, Polyneuropathy Organomegaly, Endocrinopathy or Edema, M-protein, Skin changes, Perineuritis, IgM anti-GD1b antibody syndrome (occasional).

*Toxins*: Diphtheria, Buckthorn, Hexachlorophene, Sodium Cyanate, Tellurium.

*Drugs*: Predominantly demyelinating: Chloroquine, FK506 (Tacrolimus), Perhexiline, Procainamide, Zimeldine; Mixed demyelinating & axonal: Amiodarone, Eosinophilia-Myalgia syndrome, Gold, Suramin, Taxol.

*Hereditary:* Carbohydrate-deficient glycoprotein, Cataracts & Facial dysmorphism, Cockayne's syndrome, Congenital hypomyelinating, Congenital muscular dystrophy: Merosin deficient, Farber's disease (Lipogranulomatosis), HMSN & CMT, Dominant: IA, IB, III, HNPP, EGR2, Thermosensitive , Recessive: III (Dejerine-Sottas); 4A; 4B; 4B2; 4C; 4D (LOM); 4E; 4F; HMSN-R; CNS, X-linked: IX, Krabbe, Marinesco-Sjögren, Metachromatic Leukodystrophy, Niemann-Pick, Pelizaeus-Merzbacher (PLP), Refsum, Prion protein (PrP27-30): Glu200Lys mutation, Creutzfeld-Jakob disease, Mouse model: Prion over expression, Salla disease, SOX10, Tenascin-XA, Uneven packing of peripheral myelin sheaths, Ehlers-Danlos phenotype.

*Metabolic (unusual)*: Diabetes (due to concurrent CIDP), Hypothyroidism, Hepatic disorders.

Mitochondrial: MNGIE Syndrome, Myopathy & external ophthalmoplegia, neuropathy, Gastro-Intestinal Encephalopathy, NARP Syndrome, Neuropathy, Ataxia, Retinitis, Pigmentosa.

*Infections:* Creutzfeld-Jakob disease, Diphtheria, HIV: Associated CIDP, Leprosy: Lepromatous; Mixed axonal-demyelinating; Colonized Schwann cells, Variant Creutzfeld-Jakob disease.

Multiple Sclerosis (MS) is an inflammatory demyelinating disease of the central nervous system (CNS) that takes a relapsing-remitting or a progressive course. MS is not the only demyelinating disease. Its counterpart in the peripheral nervous system (PNS) is chronic inflammatory demyelinating polyradiculoneuropathy (CIDP). In addition, there are acute, monophasic disorders, such as the inflammatory demyelinating polyradiculoneuropathy termed Guillain-Barré syndrome (GBS) in the PNS, and acute disseminated encephalomyelitis (ADEM) in the CNS. Both MS and GBS are heterogeneous syndromes. In MS different exogenous assaults together with genetic factors can result in a disease course that finally fulfils the diagnostic criteria. In both diseases, axonal damage can add to a primarily demyelinating lesion and cause permanent neurologic deficits.

MS is the most common of the above demyelinating diseases. It is characterized as an autoimmune disorder, in which leukocytes of the immune system launch an attack on the white matter of the central nervous system (CNS). The grey matter may also be involved. Although the precise etiology of MS is not known, contributing factors may include genetic, bacterial and viral infection. In its classic manifestation (85% of all cases), it is characterized by alternating relapsing/remitting phases, which correspond to episodes of neurologic dysfunction lasting several weeks followed by substantial or complete recovery (Noseworthy, 1999). Periods of remission grow shorter over time. Many patients then enter a final disease phase characterized by gradual loss of neurologic function with partial or no recovery. This is termed secondary progressive MS. A small proportion (∼15% of all MS patients) suffers a gradual and uninterrupted decline in neurologic function following onset of the disease (primary progressive MS). There is currently no clear curative treatment for the severest forms of MS, which are generally fatal.

The basic hallmark of MS is the demyelinated plaque with reactive glial scar formation, seen in the white matter tracts of the brain and spinal cord. Demyelination is linked to functional reduction or blockage in neuronal impulse conduction.

Molecular mechanisms underlying MS pathogenesis appear to stem from genetic and environmental factors, including viral and bacterial infections. These mechanisms promote increased migration of T lymphocytes and macrophages across the blood-brain barrier and into CNS tissue.

Demyelination is caused by attacks on myelin by activated macrophages and microglia, as well as damage to myelinating cells stemming from Fas-ligand signaling (or other members of the TNF-R superfamily) and complement- or antibody-mediated cytotoxicity. Therefore, demyelination occurs through both a direct attack on the myelin sheaths as well as elimination of the cells that produce and maintain myelin.

Genetic and environmental elements lead to an increased influx of inflammatory cells across the blood-brain barrier. This results in the increased migration of autoreactive T lymphocytes and macrophages into CNS tissue. Cytokine secretion by T cells activates antigen-presenting cells (APCs). When autoreactive T cells in the context of MHC class II molecules on APCs encounter putative 'MS antigens', often protein constituents of the myelin sheath, they may become activated. Several subsequent mechanisms can then act to damage oligodendrocytes and myelin. Complement- and antibody-mediated cytotoxicity may cause the majority of damage in some patients, while Fas-ligand signaling, and release of pro-inflammatory cytokines like TNF-α by CD4+ T cells may attack white matter in others. Activated macrophages may also play a role through enhanced phagocytosis and factor secretion. This causes widespread demyelination and subsequent loss of conduction efficiency among the axons of the CNS. Subsequent repair mechanisms can, however, give rise to remyelination once the inflammatory process is resolved. The remyelinated axons of MS patients are recognized pathologically by the thin appearance of the sheaths around the remyelinated axons. Additional sodium channels and an abnormal repertoire of ions channels, are often found inserted into the demyelinated axonal membrane, trying to compensate for the loss of conduction efficiency. These aberrant patterns of expression suggest that MS may also include a channelopathy. Oligodendroglial precursors may enhance remyelination in MS lesions.

The oligodendrocyte performs a multitude of functions related to its production and maintenance of the myelin sheath. This provides insulation, support and conductance enhancement for the axons of multiple neurons. A single oligodendrocyte may myelinate up to 50 different axons. Myelination is restricted only to certain, large diameter axons; dendrites and other cell processes, such as those of astrocytes, remain unmyelinated. Axons appear to exert control over the number of myelinating oligodendrocytes, since axonal transection in the paradigm of the rat optic nerve inhibits myelin renewal and oligodendrocyte precursor production (reviewed in Barres and Raff (Barres and Raff, 1999)). Oligodendrocyte proliferation and migration may be stimulated by factors released from axons during development. In this manner, the numbers of oligodendrocytes and axons are carefully matched within the CNS.

Oligodendrocytes, the perineuronal support cells of the CNS, myelinate axonal tracts and serve to enhance impulse transduction. They play roles in axonal survival and function. Note that an oligodendrocyte extends only one process to each axon it myelinates.

The multilamellar myelin sheath is a specialized domain of the oligodendroglial cell plasma membrane, rich in lipid and low in protein. It serves to support axons and improve the efficiency of electrical signal conduction in the CNS by preventing the charge from bleeding off into the surrounding tissue. The nodes of Ranvier are the sites in the sheath along the axon where saltatory conductance occurs.

In the adult brain, oligodendrocytes develop from as yet poorly defined precursor cells in the subventricular zone of the brain and spinal cord (Nait-Oumesmar *et al*., 1999). These precursors are proliferative and express myelin transcripts and proteins, first emerging in the ventral region of the embryonic spinal cord several weeks before myelination (Hajihosseini et al., 1996). The process of myelination occurs in the post-natal brain. During post-natal development, these precursors migrate to the neuron tracts that are to be myelinated.

Oligodendrocytes differentiate from mitotically active, migratory precursor cells. Once these cells have become post-mitotic, they transcribe and translate genes encoding myelin-specific proteins. The elaboration of the myelin sheath wrapping the axon is brought about by direct contact between the processes of the mature oligodendrocyte and the axon itself. CNS axon ensheathment is completed by compaction of the myelin sheath, which in its final form resembles a liquid crystal containing macromolecules in complex formation (Scherer, 1997). Promotion of myelination demands consideration of the precise stoichiometric relationship between the individual structural proteins of the myelin sheath, since increasing or decreasing the amount of one component could result in perturbation of the entire sheath structure.

The inability of oligodendrocytes to sustain repair of demyelinated axons contributes to the cumulative neurologic dysfunction characterizing MS. Promotion of remyelination in MS patients could protect axonal loss and thus limit the progression in disability associated with the death of axons in the CNS.

The demyelinating phenotype of MS led to extensive studies on the nature of the active MS lesion. Naked axons and the absence of myelinating oligodendrocytes indicated the disruption of normal myelin and aberrations in the remyelinating process associated with MS. About 40% of MS lesions were shown to exhibit evidence of abortive remyelination, especially in the early phases of the disease (Prineas et al., 1993). This presents the realistic prospect that developing strategies for promoting myelin repair could prevent permanent nervous system damage. Success probability is particularly high in younger CNS lesions, where early remyelination has already been shown to take place. However, the myelinating or remyelinating oligodendrocyte is a cell under extreme metabolic stress, which under pressure of even minor additional insults can be irreversibly damaged (Scolding and Lassmann, 1996). This decreases the probability of spontaneous repair in an active MS lesion, where inflammation and other detriments pose obstacles to remyelination. Strategies promoting myelin repair may thus stack the odds further in favor of remyelination and axonal protection in active MS lesions.

The adult human CNS has been shown to contain oligodendrocyte precursor cells that are capable of proliferating, and which could mature into myelinating oligodendrocytes. In addition, it appears that the endogenous oligodendrocyte precursor populations adjacent to MS lesions are depleted during the chronic phases of the disease, due to inhibition of these precursors' ability to proliferate and differentiate (Wolswijk, 1998). Such precursor cells are generally quiescent in the environment of a chronic MS lesion, preventing them from actively contributing to remyelination. The situation in chronic MS lesions could therefore involve factors that hamper oligodendroglial regeneration or lack factors necessary for the stimulation of the oligodendrocyte precursor cell population (Wolswijk, 1998).

Evidence for beneficial effects of remyelination in demyelinating disorders such as MS is provided by the studies performed with glial growth factors as treatments in animal models of the disease. Glial growth factor 2 (neuregulin/GGF-2), a CNS growth factor known to promote oligodendrocyte proliferation and survival, was shown to delay disease onset, reduce clinical severity and decrease relapse frequency in the EAE murine model of MS (Marchionni et al., 1999). Neuregulin was shown to have a beneficial effect on mature oligodendrocyte survival and is produced by axons (Fernandez et al., 2000).

Other growth factors, including platelet-derived growth factor (PDGF) and IGF-1, have been demonstrated to promote remyelination and have therapeutic effects in EAE models (reviewed in Dubois-Dalcq and Murray, 2000). The success achieved with the stimulation of remyelination, through inducing cells of the oligodendrocyte lineage to proliferate and/or differentiate, indicates that prospects for remyelination as a therapeutic strategy for MS are favorable. It would also be important to identify molecules that inhibit myelin synthesis, since these could lower the effectiveness of repair strategies such as oligodendroglial cell transplantation in MS.

The process of remyelination could work in concert with anti-inflammatory pathways to repair damage and protect axons from transection and death.

Oligodendrocytes may be induced to remyelinate axonal tracts in the CNS, thereby contributing to amelioration of the disease condition. Remyelination enhancement would counteract the previous destruction caused by the invasion of immune system cells into CNS tissue and their attack on myelin sheaths.

Schwann cells are peripheral glia cells providing a supportive role in the peripheral nervous system and belong to the satellite cells. Schwann cells wrap individually around the shaft of peripheral axons, forming a layer or myelin sheath along segments of the axon. Schwann cells are composed primarily of lipids or fats; the fat serves as an insulator thereby speeding the transmission rate of action potentials along the axon. Although Schwann cells are distinct from their CNS counterparts, they have many similarities in terms of structure and biology.

Schwann cells also participate in the process of neuronal regeneration in the peripheral nervous system. When an axon is dying, the Schwann cells surrounding it aid in its digestion. This leaves an empty channel formed by successive Schwann cells, through which a new axon may grow from a severed end at a rate of 3-4 millimeters a day.

Neuropathies are usually selective as to the type of PNS neuron affected (e.g. sensory versus autonomic) and indeed also to the subtype of neurons (small versus large). Axotomy of peripheral nerves is the most commonly used animal model for appraising the neuroprotective effects of neurotrophic factors. Traumatic nerve injury, plexus lesions and root lesions are a serious complication of accidents. In addition, pressure on peripheral nerve that can cause myelin damage frequently seen in disorders such as carpal tunnel syndrome or is associated with spinal column orthopedic complications. Axotomy produces phenomena, like cell death, reduced axonal conduction velocity, and altered neurotransmitter levels in damaged neurons. Crush lesions allow for regeneration, an additional process of interest in relation to neuropathic states (McMahon and Priestley, 1995).

Neuro-inflammation is a common feature to most neurologic diseases. Many stimuli are triggering neuro-inflammation, which can either be induced by neuronal or oligodendroglial suffering, or be a consequence of a trauma, of a central or peripheral nerve damage or of a viral or bacterial infection. The main consequences of neuro-inflammation are (i) secretion of various inflammatory chemokines by astrocytes; and (ii) recruitment of additional leukocytes, which will further stimulate astrocytes. In chronic neurodegenerative diseases such as multiple sclerosis (MS), Alzheimer disease (AD) or amyotrophic lateral sclerosis (ALS), the presence of persistent neuro-inflammation is though to participate to the progression of the disease. Neurologic diseases associated with neuro-inflammation can also be referred to as neurological inflammatory diseases.

IL-17F is a member of the IL-17 family of cytokines. It was independently discovered as novel cytokine ML-1 (Kawaguchi et al., 2001) or IL-17F (Hymowitz et al., 2001; Stames et al., 2001).

The IL-17 family is reviewed by Moseley et al. (Moseley et al., 2003) and Aggarwal and Gurney (Aggarwal and Gurney, 2002).

Stames et al. (2001) isolated an IL-17F cDNA encoding a 153-amino acid protein that shares 40% homology with IL-17. In addition to the 2 invariant disulfide bonds found in other members of the IL-17 family, IL-17F contains a signal peptide with an extra cys-cys pair that could function as an extra disulfide bond. IL-17F dimerizes in a parallel manner like neurotrophins, and features an unusually large cavity on its surface and it was demonstrated that it belongs to the cysteine-knot growth factor family (Hymowitz et al., 2001). IL-17F is produced by activated T cells (Kawaguchi et al., 2001; Starnes et al., 2001) and it has also been demonstrated in activated monocytes (Starnes et al., 2001). It can be induced by IL-23 (Aggarwal et al., 2003).

ML-1 was able to induce the expression of IL-6, IL-8 and ICAM-1 (intracellular adhesion molecule-1) in primary bronchial epithelial cells (PBECs) (Kawaguchi et al., 2001) via the activation of ERK1/2 (Kawaguchi et al., 2002). ML-1 was shown to induce C-X-C chemokines GROα and ENA-78 via the activation of the Raf1-MEK-ERK1/2 pathway suggesting its potential role of in the pathogenesis of airway inflammatory diseases (Kawaguchi et al., 2003).

Functional analysis by Stames et al. (Starnes et al., 2001) indicated that IL-17F induces the expression of TGFbeta and inhibits endothelial cell angiogenesis but has no effect on hematopoiesis or lymphocyte migration. They proposed that IL-17F may provide general regulation of the immune response by governing the expression of critical cytokines that have much more active stimulatory effects.

Hurst et al. (Hurst et al., 2002) suggested an in vivo role of human IL-17F in recruiting neutrophils into the pulmonary mucosa in mice after adenoviral transfer further suggesting the potential role of ML-1 in the pathogenesis of neutrophilic inflammation.

At present, the receptor(s) for IL-17F is/are not strictly identified. IL-17F does not bind with high affinity to the purified extracellular domain of either IL-17R or IL-17Rh1 (Hymowitz et al., 2001).

IL-17F nucleotide and protein sequences were disclosed in Genetics institute International patent application WO97/04097. In WO0146420, the use of IL-17 homologs, their agonists, antagonists and antibodies is described for the treatment of immune disorders.

Interferons are a subclass of cytokines that exhibit anti-inflammatory, antiviral and antiproliferative activity. On the basis of biochemical and immunological properties, the naturally-occurring human interferons are grouped into three classes: interferon alpha (leukocyte), interferon beta (fibroblast) and interferon gamma (immune). Alpha-interferon is currently approved in the United States and other countries for the treatment of hairy cell leukemia, venereal warts, Kaposi's Sarcoma (a cancer commonly afflicting patients suffering from Acquired Immune Deficiency Syndrome (AIDS)), and chronic non-A, non-B hepatitis.

Further, interferons (IFNs) are glycoproteins produced by the body in response to a viral infection. They inhibit the multiplication of viruses in protected cells. Consisting of a lower molecular weight protein, IFNs are remarkably non specific in their action, i.e. IFN induced in response to one virus is effective against a broad range of other viruses. They are however species-specific, i.e. IFN produced by one species will only stimulate antiviral activity in cells of the same or a closely related species. IFNs were the first group of cytokines to be exploited for their potential antitumor and antiviral activities.

The three major IFNs are referred to as IFN-α, IFN-β and IFN-γ. Such main kinds of IFNs were initially classified according to their cells of origin (leukocyte, fibroblast or T cell). However, it became clear that several types may be produced by one cell. Hence leukocyte IFN is now called IFN-α, fibroblast IFN is IFN-β and T cell IFN is IFN-γ. There is also a fourth type of IFN, lymphoblastoid IFN, produced in the "Namalwa" cell line (derived from Burkitt's lymphoma), which seems to produce a mixture of both leukocyte and fibroblast IFN.

The Interferon unit has been reported as a measure of IFN activity defined (somewhat arbitrarily) as the amount necessary to protect 50% of the cells against viral damage.

Every class of IFN contains several distinct types. IFN-β and IFN-γ are each the product of a single gene. The differences between individual types seem to be mainly due to variations in glycosylation.

IFNs-α are the most diverse group, containing about 15 types. There is a cluster of IFN-α genes on chromosome 9, containing at least 23 members, of which 15 are active and transcribed. Mature IFNs-α are not glycosylated.

IFNs-α and IFN-β are all the same length (165 or 166 amino acids) with similar biological activities. IFNs-γ are 146 amino acids in length, and resemble the α and β classes less closely. Only IFNs-γ can activate macrophages or induce the maturation of killer T cells. In effect, these new types of therapeutic agents can be called biologic response modifiers (BRMs), because they have an effect on the response of the organism to the tumor, affecting recognition via immunomodulation.

In particular, human fibroblast interferon (IFN-β) has antiviral activity and can also stimulate natural killer cells against neoplastic cells. It is a polypeptide of about 20,000 Da induced by viruses and double-stranded RNAs. From the nucleotide sequence of the gene for fibroblast interferon, cloned by recombinant DNA technology, Derynk et al. (Derynck et al., 1980) deduced the complete amino acid sequence of the protein. It is 166 amino acid long.

Shepard et al. (Shepard et al., 1981) described a mutation at base 842 (Cys → Tyr at position 141) that abolished its anti-viral activity, and a variant clone with a deletion of nucleotides 1119-1121.

Mark et al. (Mark et al., 1984) inserted an artificial mutation by replacing base 469 (T) with (A) causing an amino acid switch from Cys → Ser at position 17. The resulting IFN-β was reported to be as active as the 'native' IFN-β and stable during long-term storage (-70°C).

Rebif® (recombinant human Interferon-β) is a recent development in interferon therapy for multiple sclerosis (MS) and represents a significant advance in treatment. Rebif® is interferon(IFN)-beta 1a, produced from mammalian cell lines and virtually identical to the naturally occurring human molecule.

The mechanisms by which IFNs exert their effects are not completely understood. However, in most cases they act by affecting the induction or transcription of certain genes, thus affecting the immune system. In vitro studies have shown that IFNs are capable of inducing or suppressing about 20 gene products

The PRISMS study has established the efficacy of Interferon beta-1a given subcutaneously three times per week in the treatment of Relapsing-Remitting Multiple Sclerosis (RR-MS). This study showed that Interferon beta-1a can have a positive effect on the long-term course of MS by reducing the number and severity of relapses and reducing the burden of the disease and disease activity as measured by MRI (Anonymous, 1998).

Osteopontin (OPN) is a highly phosphorylated sialoprotein that is a prominent component of the mineralized extracellular matrices of bones and teeth. OPN is characterized by the presence of a polyaspartic acid sequence and sites of Ser/Thr phosphorylation that mediate hydroxyapatite binding, and a highly conserved RGD motif that mediates cell attachment/signaling. Osteopontin inhibitors have been said to be useful for treatment of infections, immune disorders and diseases, autoimmune disorders, including MS, various immunodeficiencies, and cancer, WO00/63241. The use of Osteopontin or of an agonist of osteopontin activity, is claimed in WO02/92122 for the manufacture of a medicament for the treatment and/or prevention of a neurologic disease.

Clusterin is an extracellular protein that is also known as Apolipoprotein J, SGP-2, TRPM-2 and SP-40,40. It has a nearly ubiquitous tissue distribution and many names have been given to it according to the source where it was purified (reviewed in Trougakos and Gonos (Trougakos and Gonos, 2002), Jones and Jomary (Jones and Jomary, 2002)). A neuroprotective role of clusterin in Alzheimer's disease been suggested by Giannakopoulos et al. (Giannakopoulos et al., 1998).

The treatment and/or prevention of neurologic disease with IL-17F has not yet been considered in the art.

Citation of any document herein is not intended as an admission that such document is pertinent prior art, or considered material to the patentability of any claim of the present application. Any statement as to content or a date of any document is based on the information available to applicants at the time of filing and does not constitute an admission as to the correctness of such statement.

### SUMMARY OF THE INVENTION

It is the object of the present invention to provide a novel means for the treatment and/or prevention of a neurologic disease.

The invention is based on the finding that the protein IL-17F promotes glial cell proliferation and differentiation, thus promoting myelination and regeneration of nerves. IL-17F can also promotes neuronal survival and/or differentiation. In accordance with the present invention, it has further been found that IL-17F has beneficial effects in animal models of peripheral neuropathy, multiple sclerosis as well as inflammation and neuro-inflammation.

Therefore, the present invention relates to the use of IL-17F, in a neurologic disease, such as traumatic nerve injury, stroke, demyelinating diseases of the CNS or PNS, neuropathies and neurodegenerative diseases.

In accordance with the present invention, IL-17F may also be used in combination with an interferon or osteopontin or clusterin for treatment and/or prevention of neurologic diseases. The use of nucleic acid molecules, expression vectors comprising IL-17F, and of cells expressing IL-17F, for treatment and/or prevention of a neurologic disease is also described.

The description further provides pharmaceutical compositions comprising IL-17F and an interferon or osteopontin or clusterin optionally together with one or more pharmaceutically acceptable excipients.

### BRIEF DESCRIPTION OF THE DRAWINGS

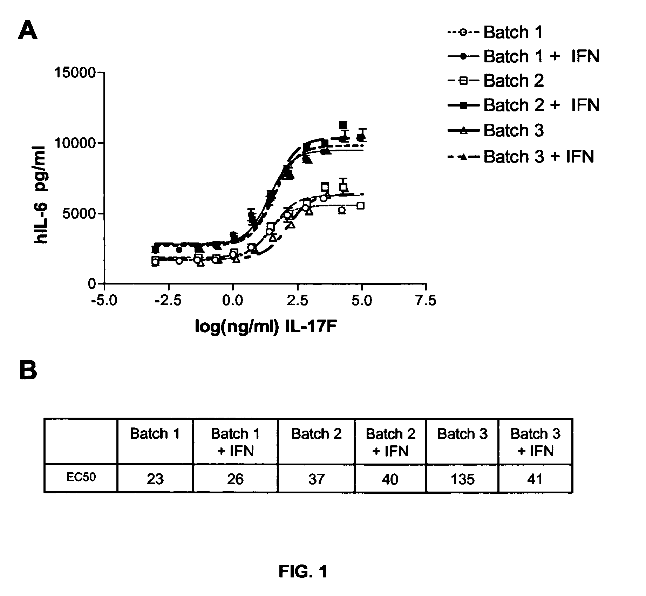

**Fig. 1** **A**. shows the results of a bioassay used to compare the bioactivity of different batches of IL-17F in the absence (Batch 1, 2, 3) or presence of rhIFNgamma (Batch 1 + IFN, Batch 2 + IFN, Batch 3 + IFN). hIL-6 release by U373 cells (5000 cells/well) plated with serial dilutions of IL-17F (log ng/ml), with or without a constant concentration of rhIFNgamma (1000 U/ml), for 24h is shown in pg/ml. **B**. The EC50 values (ng/ml) for the three batches in the presence or absence of rhIFNgamma are shown.

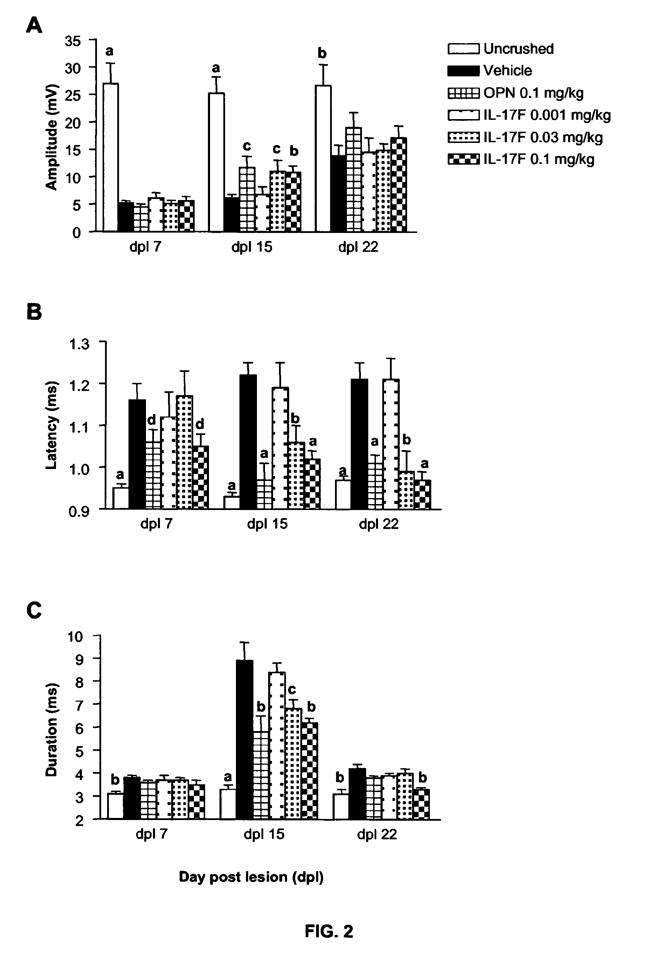

**Fig. 2** shows the electrophysiological recordings of mice, after a sciatic nerve crush, treated with Vehicle (PBS/20% glycerol/0.02% BSA), 0,001 mg/kg or 0,03 mg/kg or 0.1 mg/kg s.c. of IL-17F, 0.1 mg/kg of a reference (positive) control compound (Osteopontin; OPN). The "uncrushed" values are registered on the contralateral side of the animals. Recordings were performed at day 7, 15 and 22 post lesion (dpl).
   **2.A** represents the amplitude in millivolt (mV) of the compound muscle action potential.
   **2.B** shows the latency in milliseconds (ms) of the compound muscle action potential.
   **2.C** shows the duration in milliseconds (ms) of the compound muscle action potential.
   Statistical analyses (ANOVA followed by the Dunnett's post-test) were performed against the Vehicle group. The level of significance was set at a: p < 0.001; b: p < 0.01; c: p < 0.05; d: p < 0.1. The results were expressed as mean ± standard error of the mean (s.e.m.).

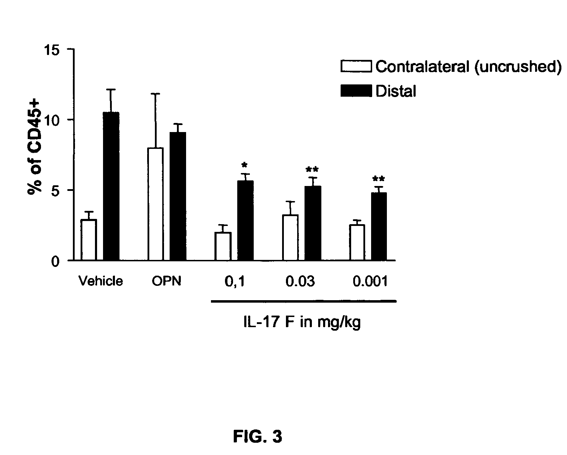

**Fig. 3** Shows leukocyte infiltration in percent of CD45 positive cells (%CD45+), as assessed by flow cytometry, on the distal part of crushed sciatic nerve and on the contralateral (uncrushed) side of mice treated with Vehicle (PBS/20% glycerol/0.02% BSA), 0,001 mg/kg or 0,03 mg/kg or 0.1mg/kg of IL-17F and 0.1 mg/kg of Osteopontin (OPN). Animals were sacrificed at day 22 post lesion. Statistical analyses (ANOVA followed by a Bonferroni post-test) were performed against the Vehicle group, for the "distal" nerves. The level of significance was set at *: p < 0.05; **: p < 0.01. The results were expressed as mean ± standard error of the mean (s.e.m.).

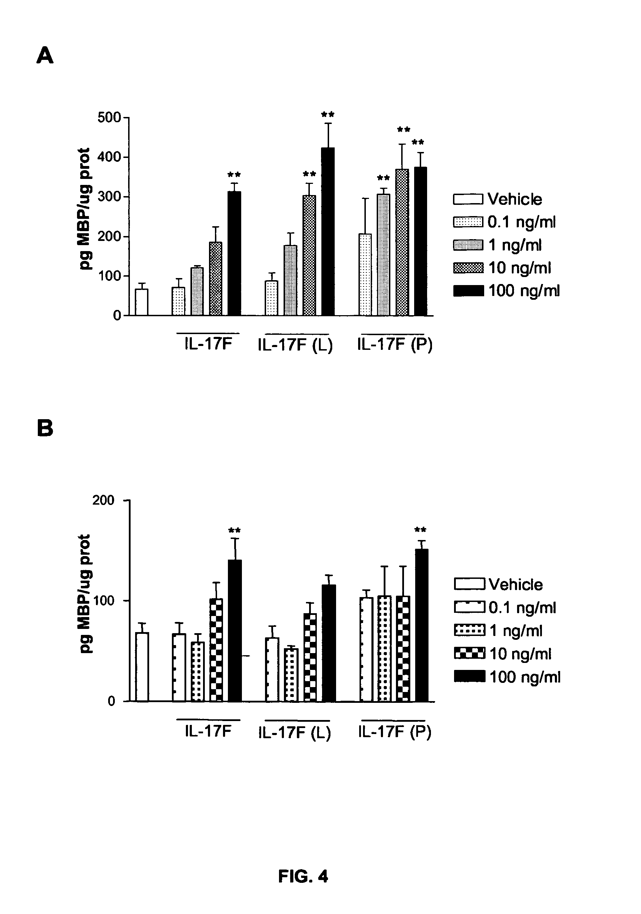

**Fig. 4** MBP content in picogram per microgram of protein (pg MBP/µg tot prot) of spinal cord mixed cultures treated with Vehicle, 0.1, 1, 10 and 100 ng/ml of IL-17F, IL-17F(L) (from Leinco) and IL-17F(P) (from Peprotech) and cultured in "1% Serum" (**4.A**) or "RPMI-B27" media (**4.B**). Measures were taken at day in vitro 11 (11DIV). Statistical analyses (ANOVA followed by the Dunnett's post-test) were performed against the control group (Vehicle). The level of significance was set at *: p < 0.05; **: p < 0.01. The results were expressed as mean ± standard error of the mean (s.e.m.).

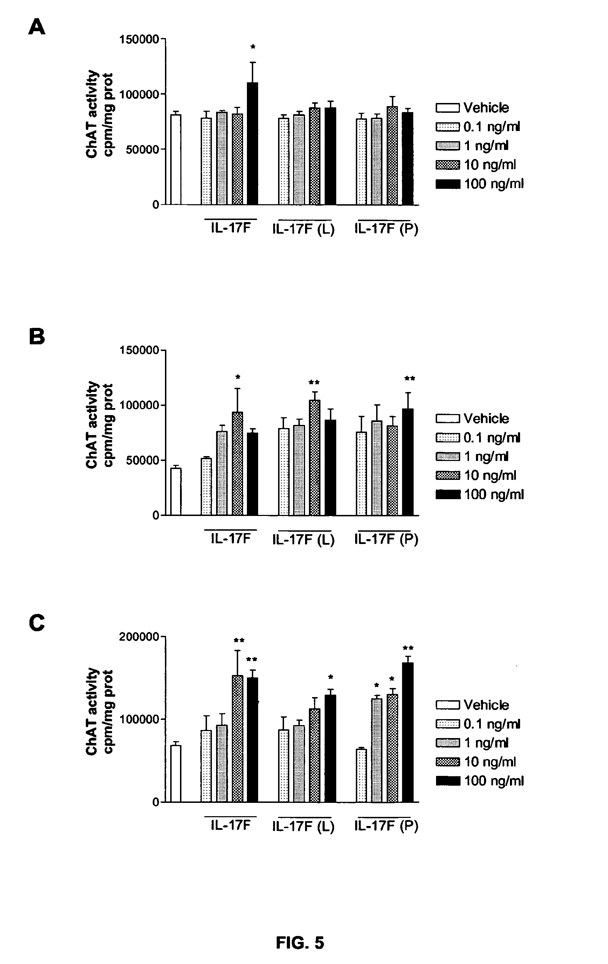

**Fig. 5** Choline acetyl transferase (ChAT) activity in counts per minute (cpm) by milligram of protein (cpm/mg prot) of spinal cord mixed cultures treated with Vehicle, 0.1, 1, 10 and 100 ng/ml of IL-17F, IL-17(L) and IL-17F(P) and cultured in "1%Serum" medium (**5.A**), "RPMI-B27" medium (**5.B**) or "RPMI-B27" medium with the addition of the antimitotic arabinoside C (AraC, 500 nM) to the cultures from day 3 onwards (**5.C**). Measures were taken at day in vitro 11 (11DIV). Statistical analyses (ANOVA followed by the Dunnett's post-test) were performed against the control group (CTRL, untreated). The level of significance was set at *: p < 0.05; **: p < 0.01. The results were expressed as mean ± standard error of the mean (s.e.m.).

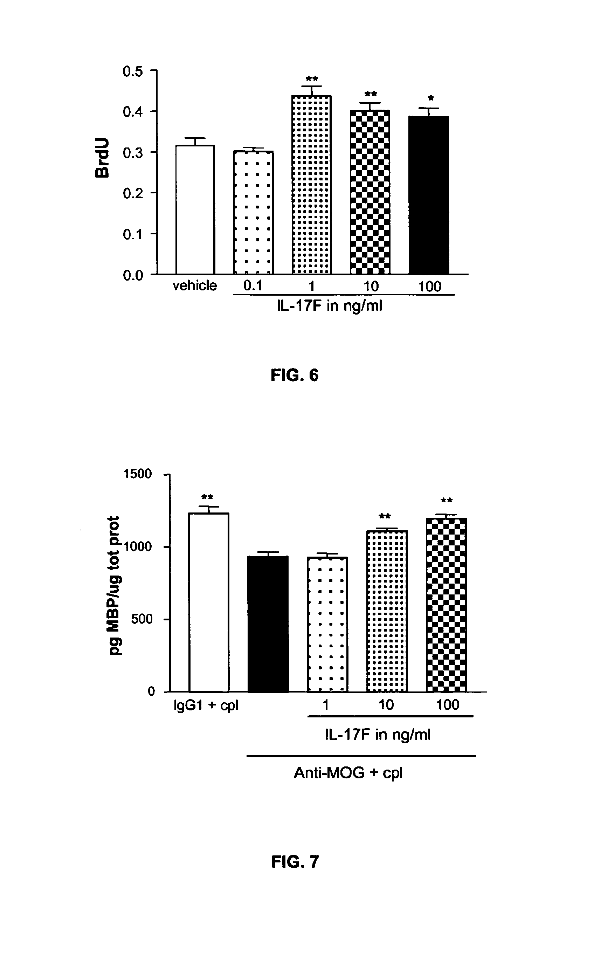

**Fig. 6** shows BrdU (Bromodeoxyuridine) incorporation (relative units, OD at 492 nm) in spinal cord mixed cultures treated with Vehicle, 0.1, 1, 10 and 100 ng/ml of IL-17F. BrdU was given to the cultures for 18 hours from day in vitro (DIV) 10 to 11. Statistical analyses (ANOVA followed by the Dunnett's post-test) were performed against the control group (CTRL, untreated). The level of significance was set at *: p < 0.05; **: p < 0.01. The results were expressed as mean ± standard error of the mean (s.e.m.).
**Fig. 7** shows the MBP content in picogram per microgram of protein (pg MBP/µg tot prot) of organotypic hippocampal slices, treated with 1, 10 and 100 ng/ml of IL-17F after demyelination induced by anti-myelin oligodendrocyte glycoprotein antibodies (anti-MOG) and baby rabbit complements (cpl). Cultures were treated for 2 days with the combination of anti-MOG antibodies and baby rabbit complement proteins (cpl), IL-17F was then given every other day during 8 days. As positive controls, cultures were treated with non-relevant isotype matching immunoglobulin IgG1 and complement (IgG1 + cpl). Statistical analyses (ANOVA followed by the Dunnett's post-test) were performed against the anti-MOG + cpl treatment. The level of significance was set at *: p < 0.05; **: p < 0.01. The results were expressed as mean ± standard error of the mean (s.e.m.)

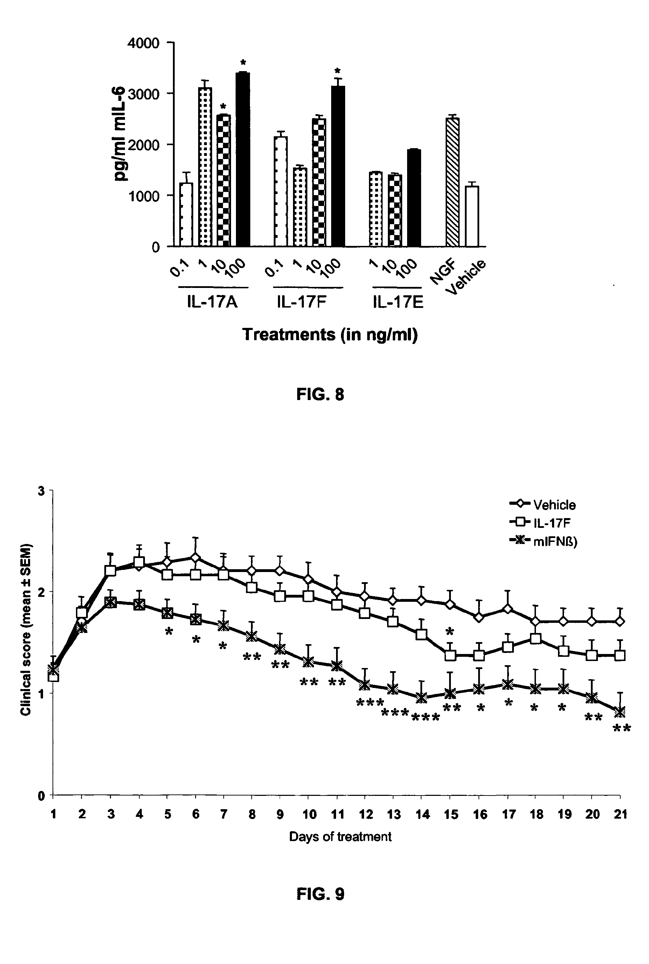

**Fig. 8** shows IL-6 release in pg/ml in mouse DRG treated with IL-17A or IL-17F at 0.1, 1, 10 or 100 ng/ml, IL-17E at 1, 10 or 100 ng/ml, 100 ng/ml of NGF as positive control or with Vehicle. The negative control consisted of untreated explants (RPMI medium alone). Statistical analyses (ANOVA followed by the Dunnett's post-test) were performed against the control group (CTRL, untreated). The level of significance was set at *: p < 0.05; **: p < 0.01. The results were expressed as mean ± standard error of the mean (s.e.m.).
**Fig. 9** effect of administration of hIL-17 at the doses of 2.5 µg/kg s.c. and mIFNβ at the dose a of 20,000 U/mouse s.c. on the clinical score of MOG-induced EAE in mice expressed as the mean (± s.e.m) score within each group. The level of significance was set at *p<0.05 **p<0.01 ***p<0.001 vs. Vehicle (one-way ANOVA followed by Fisher test).

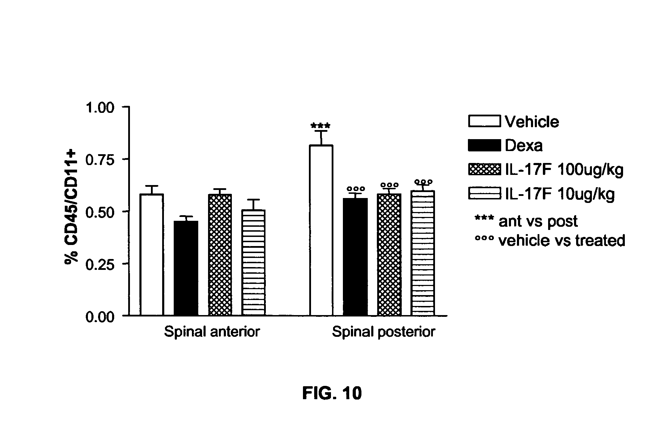

**Fig. 10** Shows leukocyte infiltration in percent of CD45/CD11 positive cells (CD45/CD11+) as assessed by flow cytometry, on the anterior (non-inflamed) and posterior parts (inflamed) of spinal cords from nerve cut operated rats treated with: vehicle, dexamethasone (0.5 mg/kg), IL-17F at 0.1 and 0.01 mg/kg. Animals were sacrificed at day 3 post lesion. Statistical analysis was performed against the control groups (***: anterior vs posterior and °°° vehicle vs treated). The level of significance was set at *:p<0.05, **: p<0.01, °°° or ***p<0.001.

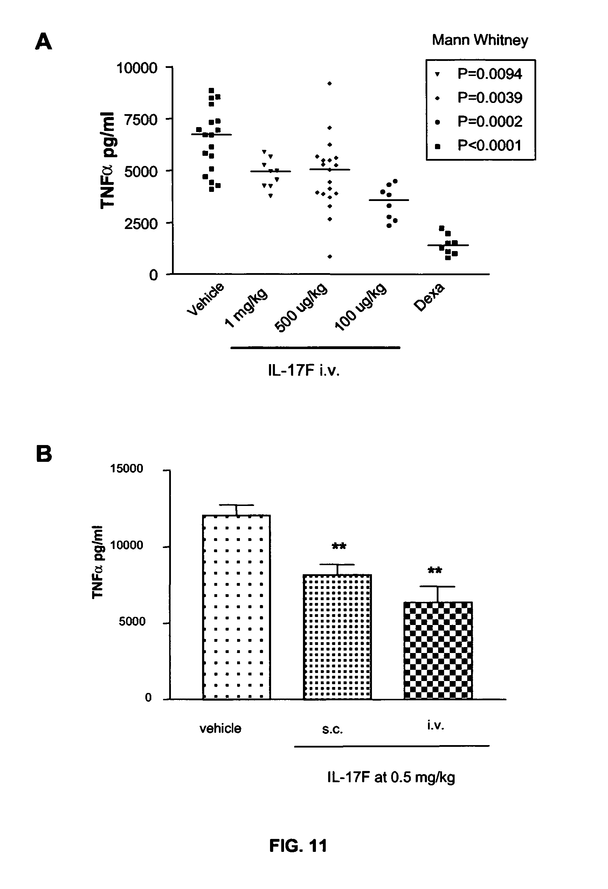

**Fig. 11** effect of IL-17F administration on TNF-α release in pg/ml in an animal model for LPS-induced TNF-α release:
   11.A. Mice were treated with Vehicle (NaCl), IL-17F at 1 mg/kg, 500 and 100 µg/kg administered by i.v. route, or dexamethasone at 0.1 mg/kg administered by s.c. route.
   11.B Animals were treated with Vehicle (NaCl) s.c. and i.v., IL-17F at 500 µg/kg administered via s.c. or i.v. routes.

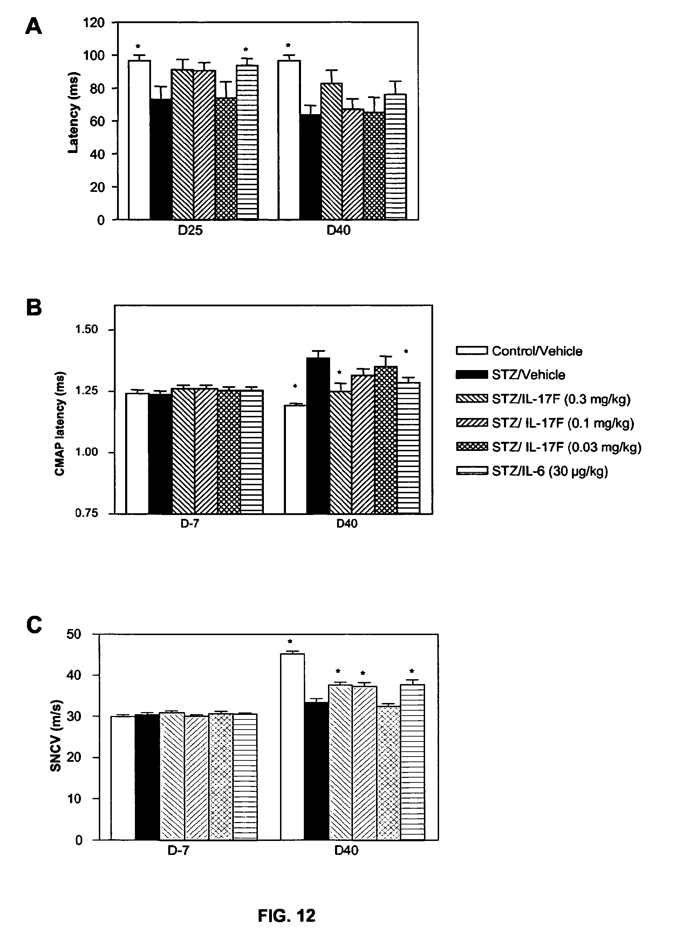

**Fig. 12** shows the effect of IL-17F in a model of diabetic neuropathy induced by the administration of streptozotocin (STZ). Rats were treated with Vehicle, IL-17F at 0.03, 0.1 or 0.3 mg/kg. IL-6 at 30µg/kg was used as a reference. The following measures were taken either 7 days before the administration of STZ (D-7) and at D40, or at D25 and D40:
   **12.A** shows the hind paw latency in milliseconds following Von Frey filament test.
   **12.B** shows the CMAP (compound muscle action potential) electrophysiological recording in milliseconds (ms).
   **12.C** shows SNCV (Sensitive nerve conduction velocity) electrophysiological recording in milliseconds (ms).

### DETAILED DESCRIPTION OF THE INVENTION

In the frame of the present invention, it has been found that administration of IL-17F has a beneficial effect in an in vivo animal model of peripheral neurological diseases. In a murine model of sciatic nerve crush induced neuropathy, all physiologic parameters relating to nerve regeneration, integrity and vitality were positively influenced by administration of IL-17F.

In addition to this, it has been shown in an autoimmune mediated demyelination model that IL-17F promotes remyelination following an induced demyelination episode.

The invention is also based on the finding that the protein IL-17F promotes glial cell proliferation and differentiation, thus promoting myelination and regeneration of nerves. IL-17F also promotes neuronal survival and/or differentiation.

In a murine model of multiple sclerosis, the MOG-induced experimental autoimmune encephalomyelitis (EAE), IL-17F given at relatively low dose was shown to improve the clinical scores of the disease.

IL-17F also revealed a beneficial effect in a model of diabetic peripheral neuropathy.

An anti-inflammatory effect of IL-17F was shown in a model of retrograde spinal cord inflammation induced by a sciatic nerve cut. Finally, IL-17F was shown to inhibit TNF-α in the LPS induced TNF-α release animal model, which is a generic model of inflammation.

The experimental evidence presented herein therefore provides for a new possibility of treating neurologic diseases, in particular those linked to neuron, glial cell function and inflammation.

The invention therefore relates to the use of IL-17F, for the manufacture of a medicament for treatment and/or prevention of neurologic diseases, as defined in the claims.

The term "IL-17F", as used herein, relates to full-length mature human IL-17F or a fragment thereof. The sequence of human IL-17F is reported herein as SEQ ID NO: 1 of the annexed sequence listing. The term "IL-17F", as used herein, further relates to any IL-17F derived from animals, such as murine, bovine, or rat IL-17F, as long as there is sufficient identity in order to maintain IL-17F activity.

Biologically active muteins and fragments, such as the naturally occurring isoforms of IL-17F are also described. Two alternatively spliced transcript variants of the gene encoding distinct isoforms of IL-17F have been reported so far (Variants 1 and 2). Variant 2 differs in the 5' end region and uses a downstream in-frame start codon, compared to variant 1. The resulting isoform 2 (amino acids 55 to 163 of SEQ ID NO: 1) has a shorter N-terminus, as compared to isoform 1 (SEQ ID NO: 1). IL-17F dimerizes in a parallel manner like neurotrophins, and features an unusually large cavity on its surface. It was demonstrated that it belongs to the cysteine-knot growth factor family (Hymowitz et al., 2001).

Isoforms, muteins, fused proteins, functional derivatives, active fractions or fragments, or circularly permutated derivatives, or salts thereof are also described. These isoforms, muteins, fused proteins or functional derivatives, active fractions or fragments, or circularly permutated derivatives retain the biological activity of IL-17F. Preferably, they have a biological activity, which is improved as compared to wild type IL-17F.

The term "agonist of IL-17F activity", as used herein, relates to a molecule stimulating or imitating IL-17F activity, such as agonistic antibodies of the IL-17F receptor, or small molecular weight agonists activating signaling through an IL-17F receptor.

The term "agonist of IL-17F activity", as used herein, also refers to agents enhancing IL-17F mediated activities, such as promotion of cell attachment to extracellular matrix components, morphogenesis of cells of the oligodendrocyte lineage into myelin producing cells, promotion of the recruitment, proliferation, differentiation or maturation of cells of the oligodendrocyte lineage (such as progenitors or precursor cells), or promotion of the protection of cells of the oligodendrocyte lineage from apoptosis and cell injury. Similar activities of IL-17F also apply to Schwann cells.

The terms "treating" and "preventing", as used herein, should be understood as preventing, inhibiting, attenuating, ameliorating or reversing one or more symptoms or cause(s) of neurologic disease, as well as symptoms, diseases or complications accompanying neurologic disease. When "treating" neurologic disease, the substances according to the invention are given after onset of the disease, "prevention" relates to administration of the substances before signs of disease can be noted in the patient.

The term "neurologic diseases", as used herein encompasses all known neurologic diseases or disorders, or injuries of the CNS or PNS, including those described in detail in the "Background of the invention".

Neurologic diseases comprise disorders linked to dysfunction of the CNS or PNS, such as diseases related to neurotransmission, headache, trauma of the head, CNS infections, neuro-ophthalmologic and cranial nerve disorders, function and dysfunction of the cerebral lobes disorders of movement, stupor and coma, demyelinating diseases, delirium and dementia, craniocervical junction abnormalities, seizure disorders, spinal cord disorders, sleep disorders, disorders of the peripheral nervous system, cerebrovascular disease, or muscular disorders. For definitions of these disorders, see e.g. The Merck Manual for Diagnosis and Therapy, Seventeenth Edition, published by Merck Research Laboratories, 1999.

In a preferred embodiment of the invention, the neurologic disease is associated with inflammation, in particular neuro-inflammation.

Preferably, the neurologic diseases of the invention are selected from the group consisting of traumatic nerve injury, stroke, demyelinating diseases of the CNS or PNS, neuropathies and neurodegenerative diseases.

Traumatic nerve injury may concern the PNS or the CNS, it may be brain or spinal cord trauma, including paraplegia, as described in the "background of the invention" above.

Stroke may be caused by hypoxia or by ischemia of the brain. It is also called cerebrovascular disease or accident. Stroke may involve loss of brain functions (neurologic deficits) caused by a loss of blood circulation to areas of the brain. Loss of blood circulation may be due to blood clots that form in the brain (thrombus), or pieces of atherosclerotic plaque or other material that travel to the brain from another location (emboli). Bleeding (hemorrhage) within the brain may cause symptoms that mimic stroke. The most common cause of a stroke is stroke secondary to atherosclerosis (cerebral thrombosis), and therefore the invention also relates to the treatment of atherosclerosis.

Peripheral Neuropathy may be related to a syndrome of sensory loss, muscle weakness and atrophy, decreased deep tendon reflexes, and vasomotor symptoms, alone or in any combination. Neuropathy may affect a single nerve (mononeuropathy), two or more nerves in separate areas (multiple mononeuropathy), or many nerves simultaneously (polyneuropathy). The axon may be primarily affected (e.g. in diabetes mellitus, Lyme disease, or uremia or with toxic agents), or the myelin sheath or Schwann cell (e.g. in acute or chronic inflammatory polyneuropathy, leukodystrophies, or Guillain-Barré syndrome). Further neuropathies, which may be treated in accordance with the present invention, may e.g. be due to lead toxicity, dapsone use, tick bite, porphyria, or Guillain-Barré syndrome, and they may primarily affect motor fibers. Others, such as those due to dorsal root ganglionitis of cancer, leprosy, AIDS, diabetes mellitus, or chronic pyridoxine intoxication, may primarily affect the dorsal root ganglia or sensory fibers, producing sensory symptoms. Cranial nerves may also be involved, such as e.g. in Guillain-Barré syndrome, Lyme disease, diabetes mellitus, and diphtheria.

Alzheimer's disease is a disorder involving deterioration in mental functions resulting from changes in brain tissue. This may include shrinking of brain tissues, primary degenerative dementia and diffuse brain atrophy. Alzheimer's disease is also called senile dementia/Alzheimer's type (SDAT).

Parkinsons's disease is a disorder of the brain including shaking and difficulty with walking, movement, and coordination. The disease is associated with damage to a part of the brain that controls muscle movement, and it is also called paralysis agitans or shaking palsy.

Huntington's Disease is an inherited, autosomal dominant neurologic disease. The genetic abnormality consists in an excess number of tandemly repeated CAG nucleotide sequences. Other diseases with CAG repeats include, for example, spinal muscular atrophies (SMA), such as Kennedy's disease, and most of the autosomal dominant cerebellar ataxias (ADCAs) that are known as spinocerebellar ataxias (SCAs) in genetic nomenclature.

Amyptrophic Lateral Sclerosis, ALS, is a disorder causing progressive loss of nervous control of voluntary muscles, including of destruction of nerve cells in the brain and spinal cord. Amyotrophic Lateral Sclerosis, also called Lou Gehrig's disease, is a disorder involving loss of the use and control of muscles.

Multiple Sclerosis (MS) is an inflammatory demyelinating disease of the central nervous system (CNS) that takes a relapsing-remitting or a progressive course. MS is not the only demyelinating disease. Its counterpart in the peripheral nervous system (PNS) is chronic inflammatory demyelinating polyradiculoneuropathy (CIDP). In addition, there are acute, monophasic disorders, such as the inflammatory demyelinating polyradiculoneuropathy termed Guillain-Barré syndrome (GBS) in the PNS, and acute disseminated encephalomyelitis (ADEM) in the CNS.

Further neurologic disorders comprise neuropathies with abnormal myelination, such as the ones listed in the "Background of the invention" above, as well as carpal tunnel syndrome. Traumatic nerve injury may be accompanied by spinal column orthopedic complications, and those are also within the diseases in accordance with the present invention.

Neurologic disorders may further be due to congenital metabolic disorders. In a preferred embodiment of the invention, the neurologic disease is therefore due to a congenital metabolic deficit.

The congenital metabolic disorders encompassed by the present invention may be e.g. phenylketonuria and other aminoacidurias, Tay-Sachs, Niemann-Pick, and Gaucher's diseases, Hurler's syndrome; Krabbe's disease and other leukodystrophies. They may affect the developing myelin sheath, mainly in the CNS.

Neurologic diseases caused by congenital metabolic disorders have also been discussed in detail in the "Background of the invention".

Less well-known neurologic diseases are also within the scope of the present invention, such as neurofibromatosis, or Multiple System Atrophy (MSA). Further disorders that may be treated in accordance with the present invention, have been described in detail in the "Background of the invention" above.

In a further preferred embodiment, the neurologic disease is a peripheral neuropathy, most preferably diabetic neuropathy. Chemotherapy associated/induced neuropathies are also preferred in accordance with the present invention.

The term "diabetic neuropathy" relates to any form of diabetic neuropathy, or to one or more symptom(s) or disorder(s) accompanying or caused by diabetic neuropathy, or complications of diabetes affecting nerves as described in detail in the "Background of the invention" above. Diabetic neuropathy may be a polyneuropathy. In diabetic polyneuropathy, many nerves are simultaneously affected. The diabetic neuropathy may also be a mononeuropathy. In focal mononeuropathy, for instance, the disease affects a single nerve, such as the oculomotor or abducens cranial nerve. It may also be multiple mononeuropathy when two or more nerves are affected in separate areas.

In a further preferred embodiment, the neurologic disorder is a demyelinating disease. Demyelinating diseases preferably comprise demyelinating conditions of the CNS, like acute disseminated encephalomyelitis (ADEM) and multiple sclerosis (MS), as well as demyelinating diseases of the peripheral nervous system (PNS). The latter comprise diseases such as chronic inflammatory demyelinating polyradiculoneuropathy (CIDP and acute, monophasic disorders, such as the inflammatory demyelinating polyradiculoneuropathy termed Guillain-Barré syndrome (GBS).

In a further preferred embodiment, the demyelinating disease is multiple sclerosis, most preferably relapsing-remitting multiple sclerosis.

In yet a further preferred embodiment, the demyelinating disease is selected from chronic inflammatory multiple sclerosis, demyelinating polyneuropathy (CIDP) and Guillain-Barré syndrome (GBS),

A further preferred embodiment of the invention relates to the treatment and/or prevention of a neurodegenerative disease. The neurodegenerative disease is selected from the group consisting of Alzheimer's disease, Parkinson's disease, Huntington's disease and ALS.

Preferably, the IL-17F is selected from a peptide, a polypeptide or a protein selected from the group consisting of:
(a) a polypeptide comprising SEQ ID NO: 1;
(b) a polypeptide comprising amino acids 11 to 163 of SEQ ID NO: 1;
(c) a polypeptide comprising amino acids 21 to 163 of SEQ ID NO: 1;
(d) a polypeptide comprising amino acids 31 to 163 of SEQ ID NO: 1;
(e) a polypeptide comprising amino acids 55 to 163 of SEQ ID NO: 1;
(i) a salt or fused protein, of any of (a) to (e).

Active fractions or fragments may comprise any portion or domain of any of the IL-17F isoforms, such as an N-terminal portion of a C-terminal portion, or any of IL-17F isoforms.

The person skilled in the art will appreciate that even smaller portions of IL-17F may be enough to exert its function, such as an active peptide comprising the essential amino acid residues required for IL-17F function.

The person skilled in the art will further appreciate that muteins, salts, isoforms, fused proteins, functional derivatives of IL-17F, active fractions or circularly permutated derivatives of IL-17F, may retain a similar, or even better, biological activity of IL-17F. The biological activity of IL-17F and muteins, isoforms, fused proteins or functional derivatives, active fractions or fragments, circularly permutated derivatives, or salts thereof, may be measured in bioassay, using a cellular system.

Preferred active fractions have an activity which is equal or better than the activity of full-length IL-17F, or which have further advantages, such as a better stability or a lower toxicity or immunogenicity, or they are easier to produce in large quantities, or easier to purify. The person skilled in the art will appreciate that muteins, active fragments and functional derivatives can be generated by cloning the corresponding cDNA in appropriate plasmids and testing them in the cellular assay, as mentioned above.

The proteins used according to the present invention may be glycosylated or non-glycosylated, they may be derived from natural sources, such as body fluids, or they may preferably be produced recombinantly. Recombinant expression may be carried out in prokaryotic expression systems such as E. coli, or in eukaryotic, such as insect cells, and preferably in mammalian expression systems, such as CHO-cells or HEK-cells. Furthermore, the proteins of the invention can be modified, extended or shortened, by removing or adding N-terminally a Methionine (Met) or aminooxypentane (AOP), as long as the neuroprotective effects are preserved.

As used herein the term "muteins" refers to analogs of IL-17F, in which one or more of the amino acid residues of a natural IL-17F are replaced by different amino acid residues, or are deleted, or one or more amino acid residues are added to the natural sequence of IL-17F, without changing considerably the activity of the resulting products as compared with the wild-type IL-17F. These muteins are prepared by known synthesis and/or by site-directed mutagenesis techniques, or any other known technique suitable therefore.

Muteins of IL-17F, or nucleic acid coding thereof, include a finite set of substantially corresponding sequences as substitution peptides or polynucleotides which can be routinely obtained by one of ordinary skill in the art, without undue experimentation, based on the teachings and guidance presented herein.

Muteins include proteins encoded by a nucleic acid, such as DNA or RNA, which hybridizes to DNA or RNA, which encodes IL-17F, in accordance with the present invention, under moderately or highly stringent conditions. The cDNA encoding IL-17F is disclosed as SEQ ID NO 2. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §§6.3 and 6.4 (1987, 1992), and Sambrook et al. (Sambrook, J. C., Fritsch, E. F., and Maniatis, T. (1989) Molecular Cloning: A Laboratory Manual, Cold Spring Harbor Laboratory Press, Cold Spring Harbor, NY).

Without limitation, examples of stringent conditions include washing conditions 12-20°C below the calculated Tm of the hybrid under study in, e.g., 2 x SSC and 0.5% SDS for 5 minutes, 2 x SSC and 0.1 % SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC. See Ausubel, supra.

In a preferred embodiment, any such mutein has at least 40% identity or homology with the sequence of SEQ ID NO: 1 of the annexed sequence listing. More preferably, it has at least 50%, at least 60%, at least 70%, at least 80% or, most preferably, at least 90% identity or homology thereto.

Identity reflects a relationship between two or more polypeptide sequences or two or more polynucleotide sequences, determined by comparing the sequences. In general, identity refers to an exact nucleotide to nucleotide or amino acid to amino acid correspondence of the two polynucleotides or two polypeptide sequences, respectively, over the length of the sequences being compared.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Methods for comparing the identity and homology of two or more sequences are well known in the art. Thus for instance, programs available in the Wisconsin Sequence Analysis Package, version 9.1 (Devereux et al., 1984), for example the programs BESTFIT and GAP, may be used to determine the % identity between two polynucleotides and the % identity and the % homology between two polypeptide sequences. BESTFIT uses the "local homology" algorithm of Smith and Waterman (Smith and Waterman, 1981) and finds the best single region of similarity between two sequences. Other programs for determining identity and/or similarity between sequences are also known in the art, for instance the BLAST family of programs (Altschul et al., 1990; Altschul et al., 1997), accessible through the home page of the NCBI at www.ncbi.nlm.nih.gov) and FASTA (Pearson, 1990; Pearson and Lipman, 1988).

Preferred changes for muteins are what are known as "conservative" substitutions. Conservative amino acid substitutions of IL-17F polypeptides, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g. under thirty, and preferably under ten, and do not remove or displace amino acids which are critical to a functional conformation, e.g. cysteine residues. Proteins and muteins produced by such deletions and/or insertions come within the purview of the present invention.

Preferably, the synonymous amino acid groups are those defined in Table I. More preferably, the synonymous amino acid groups are those defined in Table II; and most preferably the synonymous amino acid groups are those defined in Table III.

**TABLE I**

| Preferred Groups of Synonymous Amino Acids | | |
|---|---|---|
| | Amino Acid | Synonymous Group |
| | Ser | Ser, Thr, Gly, Asn |
| | Arg | Arg, Gln, Lys, Glu, His |
| | Leu | Ile, Phe, Tyr, Met, Val, Leu |
| | Pro | Gly, Ala, Thr, Pro |
| | Thr | Pro, Ser, Ala, Gly, His, Gln, Thr |
| | Ala | Gly, Thr, Pro, Ala |
| | Val | Met, Tyr, Phe, Ile, Leu, Val |
| | Gly | Ala, Thr, Pro, Ser, Gly |
| | Ile | Met, Tyr, Phe, Val, Leu, Ile |
| | Phe | Trp, Met, Tyr, Ile, Val, Leu, Phe |
| | Tyr | Trp, Met, Phe, Ile, Val, Leu, Tyr |
| | Cys | Ser, Thr, Cys |
| | His | Glu, Lys, Gln, Thr, Arg, His |
| | Gln | Glu, Lys, Asn, His, Thr, Arg, Gln |
| | Asn | Gln, Asp, Ser, Asn |
| | Lys | Glu, Gln, His, Arg, Lys |
| | Asp Glu Met Trp | Glu, Asn, Asp Asp, Lys, Asn, Gln, His, Arg, Glu Phe, Ile, Val, Leu, Met Trp |

**TABLE II**

| More Preferred Groups of Synonymous Amino Acids | | |
|---|---|---|
| | Amino Acid | Synonymous Group |
| | Ser | Ser |
| | Arg | His, Lys, Arg |
| | Leu | Leu, Ile, Phe, Met |
| | Pro | Ala, Pro |
| | Thr | Thr |
| | Ala | Pro, Ala |
| | Val | Val, Met, Ile |
| | Gly | Gly |
| | Ile | Ile, Met, Phe, Val, Leu |
| | Phe | Met, Tyr, Ile, Leu, Phe |
| | Tyr | Phe, Tyr |
| | Cys | Cys, Ser |
| | His | His, Gln, Arg |
| | Gln | Glu, Gln, His |
| | Asn | Asp, Asn |
| | Lys | Lys, Arg |
| | Asp | Asp, Asn |
| | Glu | Glu, Gln |
| | Met | Met, Phe, Ile, Val, Leu |
| | Trp | Trp |

**TABLE III**

| Most Preferred Groups of Synonymous Amino Acids | | |
|---|---|---|
| | Amino Acid | Synonymous Group |
| | Ser | Ser |
| | Arg | Arg |
| | Leu | Leu, Ile, Met |
| | Pro | Pro |
| | Thr | Thr |
| | Ala | Ala |
| | Val | Val |
| | Gly | Gly |
| | Ile | Ile, Met, Leu |
| | Phe | Phe |
| | Tyr | Tyr |
| | Cys | Cys, Ser |
| | His | His |
| | Gln | Gln |
| | Asn | Asn |
| | Lys | Lys |
| | Asp | Asp |
| | Glu | Glu |
| | Met | Met, Ile, Leu |
| | Trp | Met |

Examples of production of amino acid substitutions in proteins which can be used for obtaining muteins of IL-17F, polypeptides or proteins, for use in the present invention include any known method steps, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

The term "fused protein" refers to a polypeptide comprising IL-17F, or a mutein or fragment thereof, fused with another protein, which, e.g. has an extended residence time in body fluids. An IL-17F may thus be fused to another protein, polypeptide or the like, e.g. an immunoglobulin or a fragment thereof.

"Functional derivatives" as used herein, cover derivatives of IL-17F, and their muteins and fused proteins, which may be prepared from the functional groups which occur as side chains on the residues or the N- or C-terminal groups, by means known in the art, and are included in the invention as long as they remain pharmaceutically acceptable, i.e. they do not destroy the activity of the protein which is substantially similar to the activity of IL-17F, and do not confer toxic properties on compositions containing it.

These derivatives may, for example, include polyethylene glycol side-chains, which may mask antigenic sites and extend the residence of an IL-17F in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g alkanoyl or carbocyclic aroyl groups) or O-acyl derivatives of free hydroxyl groups (for example that of seryl or threonyl residues) formed with acyl moieties.

As "active fractions" of IL-17F, muteins and fused proteins, the present description covers any fragment or precursors of the polypeptide chain of the protein molecule alone or together with associated molecules or residues linked thereto, e.g. sugar or phosphate residues, or aggregates of the protein molecule or the sugar residues by themselves, provided said fraction has substantially similar activity to IL-17F.

The term "salts" herein refers to both salts of carboxyl groups and to acid addition salts of amino groups of IL-17F molecule or analogs thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of IL-17F relevant to the present invention, i.e., neuroprotective effect in a neurological disease.

In a preferred embodiment of the invention IL-17F is dimeric.

In a preferred embodiment of the invention, IL-17F is fused to a carrier molecule, a peptide or a protein that promotes the crossing of the blood brain barrier ("BBB"). This serves for proper targeting of the molecule to the site of action in those cases, in which the CNS is involved in the disease. Modalities for drug delivery through the BBB entail disruption of the BBB, either by osmotic means or biochemically by the use of vasoactive substances such as bradykinin. Other strategies to go through the BBB may entail the use of endogenous transport systems, including carrier-mediated transporters such as glucose and amino acid carriers; receptor-mediated transcytosis for insulin or transferrin; and active efflux transporters such as p-glycoprotein; Penetratin, a 16-mer peptide (pAntp) derived from the third helix domain of Antennapedia homeoprotein, and its derivatives. Strategies for drug delivery behind the BBB further include intracerebral implantation.

Functional derivatives of IL-17F may be conjugated to polymers in order to improve the properties of the protein, such as the stability, half-life, bioavailability, tolerance by the human body, or immunogenicity. To achieve this goal, IL-17F may be linked e.g. to Polyethlyenglycol (PEG). PEGylation may be carried out by known methods, described in WO 92/13095, for example.

Therefore, in a preferred embodiment of the present invention, IL-17F is PEGylated.

In a further preferred embodiment of the invention, the fused protein comprises an immunoglobulin (Ig) fusion. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met introduced between IL-17F sequence and the immunoglobulin sequence, for instance. The resulting fusion protein has improved properties, such as an extended residence time in body fluids (half-life), or an increased specific activity, increased expression level. The Ig fusion may also facilitate purification of the fused protein.

In a yet another preferred embodiment, IL-17F is fused to the constant region of an Ig molecule. Preferably, it is fused to heavy chain regions, like the CH2 and CH3 domains of human IgG1, for example. Other isoforms of Ig molecules are also suitable for the generation of fusion proteins according to the present invention, such as isoforms IgG₂ or IgG₄, or other Ig classes, like IgM, for example. Fusion proteins may be monomeric or multimeric, hetero- or homomultimeric. The immunoglobulin portion of the fused protein may be further modified in a way as to not activate complement binding or the complement cascade or bind to Fc-receptors.

Further fusion proteins of IL-17F may be prepared by fusing domains isolated from other proteins allowing the formation or dimers, trimers, etc. Examples for protein sequences allowing the multimerization of the polypeptides of the Invention are domains isolated from proteins such as hCG (WO 97/30161), collagen X (WO 04/33486), C4BP (WO 04/20639), Erb proteins (WO 98/02540), or coiled coil peptides (WO 01/00814).

Also described is the use of a combination of IL-17F and an immunosuppressive agent for the manufacture of a medicament for treatment and/or prevention of neurologic disorders, for simultaneous, sequential or separate use. Immunosuppressive agents may be steroids, methotrexate, cyclophosphamide, anti-leukocyte antibodies (such as CAMPATH-1), and the like.

The invention further relates to the use of a combination of IL-17F and an interferon and/or osteopontin and/or clusterin, for the manufacture of a medicament for treatment and/or prevention of neurologic disorders, for simultaneous, sequential, or separate use.

The term "interferon", as used in the present patent application, is intended to include any molecule defined as such in the literature, comprising for example any kinds of IFNs mentioned in the above section "Background of the Invention". The interferon may preferably be human, but also derived from other species, as long as the biological activity is similar to human interferons, and the molecule is not immunogenic in man.

In particular, any kinds of IFN-α, IFN-β and IFN-γ are included in the above definition. IFN-β is the preferred IFN according to the present invention.

The term "interferon-beta (IFN-β)", as used in the present invention, is intended to include human fibroblast interferon, as obtained by isolation from biological fluids or as obtained by DNA recombinant techniques from prokaryotic or eukaryotic host cells as well as its salts, functional derivatives, variants, analogs and fragments.

Of particular importance is a protein that has been derivatized or combined with a complexing agent to be long lasting. For example, PEGylated versions, as mentioned above, or proteins genetically engineered to exhibit long lasting activity in the body, can be used according to the present invention.

The term "derivatives" is intended to include only those derivatives that do not change one amino acid to another of the twenty commonly-occurring natural amino acids.

Interferons may also be conjugated to polymers in order to improve the stability of the proteins. A conjugate between Interferon β and the polyol Polyethlyenglycol (PEG) has been described in WO99/55377, for instance.

In another preferred embodiment of the invention, the interferon is Interferon-β (IFN-β), and more preferably IFN-β1a.

IL-17F is preferably used simultaneously, sequentially, or separately with the interferon.

In a preferred embodiment of the present invention, IL-17F is used in an amount of about 0.001 to 100 mg/kg of body weight, or about 0.01 to 10 mg/kg of body weight or about 9, 8, 7, 6, 5, 4, 3, 2 or 1 mg/kg of body weight or about 0.1 to1 mg/kg of body weight.

The invention further relates to the use of a nucleic acid molecule for manufacture of a medicament for the treatment and/or prevention of a neurologic disease, wherein the nucleic acid molecule comprises a nucleic acid sequence of SEQ ID NO: 2 or a nucleic acid sequence encoding a polypeptide comprising an amino acid sequence selected from the group consisting of:
(a) a polypeptide comprising SEQ ID NO: 1;
(b) a polypeptide comprising amino acids 11 to 163 of SEQ ID NO: 1;
(c) a polypeptide comprising amino acids 21 to 163 of SEQ ID NO: 1;
(d) a polypeptide comprising amino acids 31 to 163 of SEQ ID NO: 1;
(e) a polypeptide comprising amino acids 55 to 163 of SEQ ID NO: 1;

The nucleic acid may e.g. be administered as a naked nucleic acid molecule, e.g. by intramuscular injection.

It may further comprise vector sequences, such as viral sequence, useful for expression of the gene encoded by the nucleic acid molecule in the human body, preferably in the appropriate cells or tissues.

Therefore, in a preferred embodiment, the nucleic acid molecule further comprises an expression vector sequence. Expression vector sequences are well known in the art, they comprise further elements serving for expression of the gene of interest. They may comprise regulatory sequence, such as promoter and enhancer sequences, selection marker sequences, origins of multiplication, and the like. A gene therapeutic approach is thus used for treating and/or preventing the disease. Advantageously, the expression of IL-17F will then be *in situ*.

In a preferred embodiment, the expression vector is a lentiviral derived vector. Lentiviral vectors have been shown to be very efficient in the transfer of genes, in particular within the CNS. Other well established viral vectors, such as adenoviral derived vectors, may also be used according to the invention.

A targeted vector may be used in order to enhance the passage of IL-17F across the blood-brain barrier. Such vectors may target for example the transferrin receptor or other endothelial transport mechanisms.

In a preferred embodiment of the invention, the expression vector may be administered by intramuscular injection.

The use of a vector for inducing and/or enhancing the endogenous production of IL-17F in a cell normally silent for expression of IL-17F, or which expresses amounts of IL-17F which are not sufficient, are also contemplated according to the invention. The vector may comprise regulatory sequences functional in the cells desired to express IL-17F. Such regulatory sequences may be promoters or enhancers, for example. The regulatory sequence may then be introduced into the appropriate locus of the genome by homologous recombination, thus operably linking the regulatory sequence with the gene, the expression of which is required to be induced or enhanced. The technology is usually referred to as "endogenous gene activation" (EGA), and it is described e.g. in WO 91/09955.

Also described is the use of a cell that has been genetically modified to produce IL-17F in the manufacture of a medicament for the treatment and/or prevention of neurologic diseases.

Also described is a cell that has been genetically modified to produce IL-17F for manufacture of a medicament for the treatment and/or prevention of neurologic diseases. Thus, a cell therapeutic approach may be used in order to deliver the drug to the appropriate parts of the human body.

The application further describes pharmaceutical compositions, particularly useful for prevention and/or treatment of neurologic diseases, which comprise a therapeutically effective amount of IL-17F and a therapeutically effective amount of an interferon and/or osteopontin and/or clusterin optionally further a therapeutically effective amount of an immunosuppressant.

The definition of "pharmaceutically acceptable" is meant to encompass any carrier, which does not interfere with effectiveness of the biological activity of the active ingredient and that is not toxic to the host to which it is administered, or that can increase the activity. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g. in slow release formulations), intramuscular, intraperitoneal, intravenous, subcutaneous, oral, epidural, topical, intrathecal, rectal, and intranasal routes. Any other therapeutically efficacious route of administration can be used, for example absorption through epithelial or endothelial tissues or by gene therapy wherein a DNA molecule encoding the active agent is administered to the patient (e.g. via a vector), which causes the active agent to be expressed and secreted in vivo.

In addition, the protein(s) according to the invention can be administered together with other components of biologically active agents such as pharmaceutically acceptable surfactants, excipients, carriers, diluents and vehicles.

For parenteral (e.g. intravenous, subcutaneous, intramuscular) administration, the active protein(s) can be formulated as a solution, suspension, emulsion or lyophilised powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the invention can also be ameliorated by using conjugation procedures which increase the half-life of the molecule in the human body, for example linking the molecule to polyethylenglycol (PEG), as described in the PCT Patent Application WO 92/13095.

The therapeutically effective amounts of the active protein(s) will be a function of many variables, including the type of protein, the affinity of the protein, any residual cytotoxic activity exhibited by the antagonists, the route of administration, the clinical condition of the patient (including the desirability of maintaining a non-toxic level of endogenous IL-17F activity).

A "therapeutically effective amount" is such that when administered, the IL-17F exerts a beneficial effect on the neurologic disease. The dosage administered, as single or multiple doses, to an individual will vary depending upon a variety of factors, including IL-17F pharmacokinetic properties, the route of administration, patient conditions and characteristics (sex, age, body weight, health, size), extent of symptoms, concurrent treatments, frequency of treatment and the effect desired.

As mentioned above, IL-17F can preferably be used in an amount of about 0.001 to 100 mg/kg of body weight, or about 0.01 to 10 mg/kg of body weight or about 9, 8, 7, 6, 5, 4, 3, 2 or 1 mg/kg of body weight or about 0.1 to 1 mg/kg of body weight.

The route of administration, which is preferred according to the invention is administration by subcutaneous route. Intramuscular administration is further preferred according to the invention.

In further preferred embodiments, IL-17F is administered daily or every other day.

The daily doses are usually given in divided doses or in sustained release form effective to obtain the desired results. Second or subsequent administrations can be performed at a dosage which is the same, less than or greater than the initial or previous dose administered to the individual.

According to the invention, IL-17F can be administered prophylactically or therapeutically to an individual prior to, simultaneously or sequentially with other therapeutic regimens or agents (e.g. multiple drug regimens), in a therapeutically effective amount, in particular with an interferon. Active agents that are administered simultaneously with other therapeutic agents can be administered in the same or different compositions.

Reference to known method steps, conventional methods steps, known methods or conventional methods is not any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation, without departing from the general concept of the present invention. Therefore, such adaptations and modifications are intended to be within the meaning of a range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

Having now described the invention, it will be more readily understood by reference to the following examples that are provided by way of illustration and are not intended to be limiting of the present invention.

### EXAMPLES

### Abbreviations

| | |
|---|---|
| CMAP | compound muscle action potential |
| div | day in vitro |
| dpl | day post lesion |
| EMG | Electomyograph |
| i.p. | intraperitoneal |
| i.v. | intravenous |
| s.c. | subcutaneous |
| s.e.m. | standard error of the mean |
| SNCV | Sensitive nerve conduction velocity |

### EXAMPLE 1: Recombinant expression of IL-17F

### Cloning and expression

IL-17F coding sequence was amplified from Jurkat T cells and cloned into pCR4-TOPO vector using the gene bank accession number AF384857. It was then cloned into a pEAK12 vector with a tag at the C-terminus.

Tagged recombinant human IL-17F (in the following IL-17F) was expressed in HEK cells and purified as follows:
Human Embryonic Kidney 293 cells expressing the Epstein-Barr virus Nuclear Antigen (HEK293-EBNA, Invitrogen) were maintained in suspension in Ex-cell VPRO serum-free medium (seed stock, maintenance medium, JRH). On the day of transfection, cells were counted, centrifuged (low speed) and the pellet re-suspended into the desired volume of FEME medium (500 ml) supplemented with 1% FCS to yield a cell concentration of 1XE6 viable cells / ml. The cDNA was diluted at 2mg / liter volume in FEME (200 ml / liter volume). PolyEthylenelmine transfection agent (4mg/ liter volume) was then added to the cDNA solution, vortexed and incubated at room temperature for 10 minutes (generating the transfection Mix).
This transfection mix was then added to the spinner and incubated for 90 minutes in a CO₂ incubator (5% CO₂ and 37°C). Fresh FEME medium (1% FCS) was added after 90 minutes such as to double the initial spinner volume. The spinner was then incubated for 6 days. On day 6 (harvest day), spinner supernatant (500ml) was centrifuged (4°C, 400g) and placed into a pot bearing a unique identifier with plasmid number and fermentation number.

### Purification process

The 3000 ml culture medium sample containing the recombinant protein with a C-terminal tag was diluted with one volume cold buffer A (50 mM NaH₂PO₄; 600 mM NaCl; 8.7 % (w/v) glycerol, pH 7.5) to a final volume of 6000 ml. The sample was filtered through a 0.22 µm sterile filter (Millipore, 500 ml filter unit) and kept at 4°C.

The purification was performed at 4°C on the VISION workstation (Applied Biosystems). The purification procedure was composed of two sequential steps, tag affinity chromatography on a Poros 20 MC (Applied Biosystems) column charged with Ni ions (10 x 100 mm, 7.8 ml), followed by buffer exchange on a Sephadex G-25 medium (Amersham Pharmacia) gel filtration column (1,0 x 15 cm).

The first chromatography step was carried out according to manufacturer's protocol.

For the second chromatography step, the Sephadex G-25 gel-filtration column was regenerated with 2 ml of buffer D (1.137 M NaCl; 2.7 mM KCI; 1.5 mM KH₂PO₄; 8 mM Na₂HPO₄; pH 7.2), and subsequently equilibrated with 4 column volumes of buffer C (137 mM NaCl; 2.7 mM KCI; 1.5 mM KH₂PO₄; 8 mM Na₂HPO₄; 20 % (w/v) glycerol; pH 7.4). The peak fraction eluted from the metal ion-column was automatically, through the integrated sample loader on the VISION, loaded onto the Sephadex G-25 column and the protein was eluted with buffer C at a flow rate of 2 ml/min. The desalted sample was recovered in a 3.5 ml fraction. The fraction was filtered through a 0.22µm sterile centrifugation filter (Millipore), aliquoted, frozen and stored at -80° C. An aliquot of the sample was analyzed on SDS-PAGE (4-12 % NuPAGE gel; Novex) by Coomassie blue staining and Western blot with anti-tag antibodies.

Coomassie Blue staining. The NuPAGE gel was stained in a 0.1 % coomassie blue R250 staining solution (30 % methanol, 10 % acetic acid) at room temperature for 1 h and subsequently destained in 20 % methanol, 7.5 % acetic acid until the background was clear and the protein bands clearly visible (not shown).

Western blot. Following the electrophoresis the proteins were electrotransferred from the gel to a nitrocellulose membrane at 290 mA for 1 hour at 4°C. The membrane was blocked with 5 % milk powder in buffer E (137 mM NaCl; 2.7 mM KCI; 1.5 mM KH₂PO₄; 8 mM Na₂HPO₄; 0.1 % Tween 20, pH 7.4) for 1 h at room temperature, and subsequently incubated with a mixture of 2 rabbit polyclonal anti-tag antibodies (G-18 and H-15, 0.2ug/ml each; Santa Cruz) in 2.5 % milk powder in buffer E overnight at 4°C. After further 1 hour incubation at room temperature, the membrane was washed with buffer E (3 x 10 min), and then incubated with a secondary HRP-conjugated anti-rabbit antibody (DAKO, HRP 0399) diluted 1/3000 in buffer E containing 2.5 % milk powder for 2 hours at room temperature. After washing with buffer E (3 x 10 minutes), the membrane was developed with the ECL kit (Amersham) for 1 min. The membrane was subsequently exposed to a Hyperfilm (Amersham), the film developed and the Western blot image visually analyzed. Alternatively, the membranes were incubated with commercial anti-IL-17F antibodies from R&D (polyclonal: 1:500 or monoclonal 1:250), and revealed with appropriate secondary antibodies.

The protein concentration was determined using the calculated extinction coefficient at 280 nm, 14,660 M⁻¹ cm⁻¹. The protein yield was 28 and 7.8 mg, respectively for the two preparations performed.

### EXAMPLE 2: Bioassay to test IL-17F activity

### Introduction

A test was performed to check the bioactivity of IL-17F in order to compare activities of the different purified batches (Batch 1 to 3).

### Material and method

U373 glioma cells (from European Collection of Cell cultures, England) were plated in 96-well plates (5000 cells/well) in 10%FCS containing MEM, treated with serial dilutions of IL-17F with or without a constant concentration of rhIFNgamma (1000 U/ml, R&D) and incubated for 24h (37C, 10% CO2). Diluted supernatants (50 x in PBS) were tested after 24h for human IL-6 release, using a commercial ELISA (from R&D).

### Results

Fig. 1.A and B show that the different purification products have very similar activities. The addition of rhIFNgamma does not significantly alter the EC50 values, however it increases the signal to noise ratio of the assay.

As there was no significant difference between the batches, in the following no reference to the respective bach will be made.

### EXAMPLE 3: Protective effect of IL-17F on neuropathy induced by sciatic nerve crush in mice

### Introduction

The present study was carried out to evaluate nerve regeneration and remyelination in mice treated with IL-17F at different doses. In this model a positive effect of IL-17F on neuronal and axonal (sensory and motor neurons) survival and regeneration, or on myelination or macrophage inflammation, may lead to restoration of motor function. The regeneration can be measured according to the restoration of sensorimotor functions, which can be evaluated by electrophysiological recordings.

### Materials and Methods

### Animals

Thirty 8 week-old females C57bI/6 RJ mice (Elevage Janvier, Le Genest-St-Isle, France) were used. They were divided into 5 groups (n = 6): (a) vehicle nerve crush operated group; (c) nerve crush/IL-17F (0.1 mg/kg); (d) nerve crush/IL-17F (0.03 mg/kg); (e) nerve crush/IL-17F (0.001 mg/kg); (f) nerve crush/Osteopontin (0.1 mg/kg). Osteopontin (OPN) is a highly phosphorylated sialoprotein that is a prominent component of the mineralized extracellular matrices of bones and teeth. WO02092122 discloses the effects of OPN in neurological diseases.

The animals were group-housed (6 animals per cage) and maintained in a room with controlled temperature (21-22°C) and a reversed light-dark cycle (12h/12h) with food and water available *ad libitum*. All experiments were carried out in accordance with institutional guidelines.

### Lesion of the sciatic nerve

The animals were anaesthetized by inhalation of 3% Isofluran^{®} (Baxter). The right sciatic nerve was surgically exposed at mid thigh level and crushed at 5 mm proximal to the trifurcation of the sciatic nerve. The nerve was crushed twice for 30s with a haemostatic forceps (width 1.5 mm; Koenig; Strasbourg; France) with a 90 degrees rotation between each crush.

### Planning of experiments and pharmacological treatment

Electromyographical (EMG) testing was performed once before the surgery day (baseline) and each week during 3 weeks following the operation.

The day of nerve crush surgery was considered as dpl 0 (dpl = day post lesion). No test was performed during the 4 days following the crush. Body weight and survival rate were recorded every day.

From the day of nerve injury to the end of the study, IL-17F or OPN were administered daily by subcutaneous injections (s.c.) route, except on week-ends. Vehicle consisted of PBS/20% glycerol/0.02% BSA. At 22 dpl, all animals were sacrificed and sciatic nerves were dissected and dissociated to perform FACS analyses on the infiltrating cells.

### Electrophysiological recording

Electrophysiological recordings were performed using a Neuromatic 2000M electromyograph (EMG) (Dantec, Les Ulis, France). Mice were anaesthetized by inhalation of 3% Isofluran^{®} (Baxter). The normal body temperature was maintained at 30°C with a heating lamp and controlled by a contact thermometer (Quick, Bioblock Scientific, IIIkirch, France) placed on the tail.

Compound muscle action potential (CMAP) was measured in the *gastrocnemius* muscle after a single 0.2 ms stimulation of the sciatic nerve at a supramaximal intensity of 12.8 mA. The amplitude (mV), the latency (ms) and the duration (time needed for a depolarization and a repolarization session) of the action potential were measured both on the operated leg, and on the contralateral leg (=uncrushed). The amplitude and duration are indicative of the number of active motor units, while the distal latency indirectly reflects motor nerve conduction and neuromuscular transmission velocities, which depends in part on the degree of myelination.

### Flow cytometry (FACS) analyses of the leukocyte infiltration

After sacrifice of the animals at day 22 post lesion, the sciatic nerves were dissected out (distal part and contralateral), minced and incubated in collagenase (8 mg/ml) and 1% DNAse for 2h at 37C. The distal part represents the part of the sciatic nerves that go from the crush to the end of the leg, whereas the contralateral part represents the uncrushed leg. The pieces were then mechanically dissociated with a pipet and passed through a 70 micron mesh. The cells were then washed and blocked in 1%BSA, 0.01% NaN3 and rabbit IgGs (3 ug/ml) for 30 min, and incubated in phycoerthryin-conjugated mouse anti-CD45 antibodies (25 ug/ml, from R&D) for 45 min. The cells were then analyzed on FACSCalibur from Becton Dickinson. The percent of CD45 positive cells was reported on a graph, for the distal and contralateral parts, for all treatments. Four animals were taken for each treatment.

### Data analysis

Global analysis of the data was performed using one-way ANOVA. Dunnett's test was used further, and data were compared to the "vehicle" control. The level of significance was set at a: p < 0.001; b or **: p < 0.01; c or *: p < 0.05; d: p < 0.1 . The results were expressed as mean ± standard error of the mean (s.e.m.).

### Results

All animals survived the nerve crush procedures and throughout the study. The electrophysiological values of the uncrushed contraleral side were significantly different from the vehicle-crushed side at all times tested, as expected.

### Animal weight

None of the animals lost significant body weight during the study (not shown).

### Electrophysiological measurements

### Amplitude of the compound muscular action potential (Fig. 2.A):

No significant change in the CMAP amplitude throughout the study was observed in the contralateral "uncrushed" legs. In contrast, crush of the sciatic nerve induced a dramatic decrease in the amplitude of CMAP with a decrease in the Vehicle group of about 80% at dpl7 and dpl15, when compared to the respective levels of contralateral side (uncrushed). When mice were treated with IL-17F, at 0.1 mg/kg or 0.03 mg/kg, or osteopontin at 0.1 mg/kg, they demonstrated an increase (about 1.8 times) in the CMAP amplitude, as compared to the levels in untreated mice, but this effect was significant only at 15 dpl.

### Latency of the compound muscular action potential (Fig 2.B):

Muscles on the crushed side showed 1.2 times greater CMAP latency than contralateral "uncrushed" legs. In mice treated with IL-17F or osteopontin, the CMAP latency value was significantly reduced as compared to the one of Vehicle mice. At day 7, this effect could be observed after treatment with 0.1 mg/kg of IL-17F or 0.1 mg/kg of osteopontin. At dpl 15 and 22, a significant effect was obtained with 0.1 mg/kg and 0.03 mg/kg (but not with 0.001 mg/kg) of IL-17F. Statistical significance was also obtained with the reference compound osteopontin, at all days tested.

### Duration of the compound muscular action potential (Fig 2.C):

Muscles on the crushed side showed a significant extension of CMAP duration in the Vehicle group, when compared to the contralateral side, especially at dpl 15 where the duration was 2.7 times greater than in contralateral (uncrushed) legs. This duration was significantly reduced after two weeks when IL-17F was given at 0.1 or 0.03 mg/kg (but not at 0.001 mg/kg), whereas at dpl 22, only 0.1 mg/kg was still significantly different.

### Flow cytometry (FACS) analyses of the leukocyte infiltration (Fig. 3)

Three weeks after the nerve crush, the leukocyte infiltration in the distal part of the crushed sciatic nerve was high in all groups when compared to the contralateral (uncrushed) side. In animals treated with IL-17F, this infiltration was significantly reduced at all doses when compared to the Vehicle-treated animals.

### Conclusions

The nerve-crush model is a very dramatic model of peripheral neuropathy. Immediately after the nerve crush most of the fibers having a big diameter are lost, due to the mechanical injury, leading to the strong decrease in the CMAP amplitude. The CMAP latency was not immediately affected but showed an increase at 15 days due to additional degeneration of small diameter fibers by secondary, immune mediated degeneration (macrophages, granulocytes). The CMAP duration increased at dpl 7 and peaked at dpl 15.

IL-17F showed a protective effect in the nerve crush model in mice on all parameters measured. IL-17F was as effective as the reference molecule used in this study, osteopontin. Also, the leukocyte infiltration seems to be rather decreased after IL-17F treatments, when compared to vehicle treated animals.

### EXAMPLE 4: IL-17F stimulates Myelin Basic Protein (MBP) production in maturating mixed spinal cord cultures (Figs 4-6).

### Introduction

Regulation of nerve regeneration after injury or disease requires not only axonal sprouting and elongation, but also new myelin synthesis. Myelination is necessary for the normal nerve conduction and axonal protection against excitotoxicity or immunologic attacks for example. Because myelin repair is mostly a recapitulation of ontogenetic events (Capello et al., 1997; Kuhn et al., 1993), and because motoneurons are affected in a number of peripheral neuropathies, we used spinal cord mixed cultures to assess the effects of IL-17F on myelin and neuronal parameters. More precisely, the myelin basic protein (MBP) content, a protein representative of matured oligodendrocytes and Schwann cells, was monitored by ELISA. Choline acetyl transferase (ChAT) activity, an enzyme representative of the cholinergic activity of motoneneurons, was determined using an enzymatic assay. BrdU (Bromodeoxyuridine) incorporation was also quantified to evaluate IL-17F effects on proliferation.

### Materials and Methods

### Cultures

Mixed spinal cord cultures were prepared according to the protocol of Messmer-Joudrier et al. (Messmer-Joudrier et al., 1996). Briefly, spinal cords of E14 Wistar rat embryos were dissociated by an enzymatic digestion (trypsin-EDTA) followed by a mechanical dissociation using a fire polished glass pipet. Cells (2*10⁵ cells/well) were then grown in poly-L-Lysine-coated 96-well plates, in 0.5% FCS-0.5% horse serum-DMEM medium ("1% serum" medium), or in 50% RPMI-50% Neurobasal medium-0.25% FCS-0.25% horse serum-1% B27 ("RPMI-B27" medium). Treatments with IL-17F were initiated at day 0 and renewed twice a week. In some experiments, the antimitotic arabinoside C (AraC, 500 nM) was added to the cultures, from day 3 to the end. Cultures were than lysed and processed for MBP ELISA, or ChAT activity as described below. In some experiments, BrdU incorporation was performed in order to evaluate the effects of IL-17F on proliferation, and quantified with a commercial ELISA kit.

### MBP-ELISA

The capture antibody, mouse monoclonal antibody anti-MBP (1/5000; Chemicon), was diluted in PBS and incubated overnight at 4°C. The plates were blocked with PBS containing 1% BSA for 1 hour. Samples diluted in PBS were incubated for 2 hours. The detection antibody, rabbit polyclonal anti-MBP (1/300; Zymed) diluted in PBS-BSA, was incubated for 2 hours and revealed with an O-phenylenediamine Dihydrochloride (OPD) substrate after incubation with anti-rabbit horse-radish peroxidase-conjugated antibody (1/3000, Sigma; diluted in PBS-BSA, 1 hours). Values of the optic densities read at 492 nm were reported to a standard curve of MBP (InVitrogen) and then to the content of protein of each sample.

### Choline acetyl transferase (ChAT) enzymatic assay

This assay was performed following the technique of Fonnum (1969) (Fonnum, 1969). Briefly, samples were incubated in a mix of phosphate buffer (0.05M, pH 7.4), containing choline chloride (0.01 M, Sigma), NaCl 3M and bovine serum albumin (0.01%), at 37C for 20 min. Neostigmin (an acetyl choline esterase inhibitor, 0.2 mM, Sigma) and ³H-acetyl Co-A (1 uCi/ml) were also added to the mix. The reaction was stopped by adding 14 volumes of tetraphenyl boron in acetonitril (2 mg/ml, FLUKA). Tetraphenylboron complexes the choline esters formed by the activity of the choline acetyl transferase (contained in the samples) on the choline and the acetyl-CoA. These complexes being insoluble in water, a liquid cation exchange was performed using an organic scintillation fluid (Instafluor Plus, Perkin Elmer), thus only the radioactivity in the organic phase was read in the scintillation counter. For that purpose, 2.5 volumes (of the reaction product + stop solution) of phosphate buffer (0.05 M, pH 7.4) were added and 1.4 volume (of the resulting volume) of scintillation fluid was added and mixed vigorously. Results were finally expressed in counts (cpm) per ml and were divided by the protein concentration of the samples (measured with the BCA kit from Pierce).

### BrdU ELISA

BrdU incorporation and ELISA were performed according to the manufacturer's instructions (Amersham). Briefly, after an 18-hour incubation with BrdU, cells were washed, fixed and incubated with an HRP-conjugated-anti-BrdU antibody, which was then revealed with a colorimetric substrate. The product of the reaction was then read at 492 nm and relative units were reported on a graph.

### Results

Commercial recombinant IL-17F from Leinco (IL-17F (L)) and Peprotech (IL-17F (P)), were tested to compare the activity of our protein, produced in HEK cells and carrying a tag, with the activity of IL-17F produced in E. coli, without a tag. Fig. 4 shows that MBP content was significantly increased by IL-17F treatments, with IL-17F from all sources, even if significance was achieved at somewhat different levels (between 10 and 100 ng/ml). A dose-response was obtained in "1% serum" medium, (Fig. 4A) which was a medium that favoured all cell types, whereas the "RPMI-B27" medium favoured mainly the neurons. Consequently, the increased MBP content induced by IL-17F in cultures grown in RPMI-B27 was not as pronounced (Fig. 4B). As a result of the use of these different media, a positive effect of IL-17F was observed on ChAT activity (Fig.5 A, B, C), especially in cultures grown in RPMI-B27 (Fig. 5B). The increase in ChAT activity was even more obvious in cultures that were treated with the antimitotic AraC (Fig. 5C), which eliminated proliferating cells (i.e. glial cells, since neurons did not proliferate any more in such cultures). This increased ChAT activity could have reflected either increased differentiation or increased survival of neurons.

Taken together, these results suggested that IL-17F increased MBP by inducing proliferation of oligodendrocytes progenitors, whereas the effect of IL-17F on neurons could have been direct (on differentiation or survival), or indirect (by producing more myelin, by providing therefore more trophic support for the neurons).

To further confirm the induction of proliferation by IL-17F, BrdU incorporation was tested at different time points following IL-17F treatments using a commercial ELISA kit. It was found that IL-17F did indeed increase the incorporation of BrdU (Fig. 6, shown at 11DIV). TUNEL stainings indicated that there was no increased apoptosis in treated cultures, and LDH release in the supernatants was also tested. None of these parameters indicated that IL-17F was toxic for the cultures.

### Conclusion

It has been shown that IL-17F increases MBP content and ChAT level in mixed spinal cord cultures from rat embryos.

### EXAMPLE 5: IL-17F increases remyelination of hippocampal slices after demyelination induced by anti-MOG antibody and baby rabbit complement proteins (Fig.7).

### Introduction

Breakdown of myelin, a characteristic of chronic inflammatory multiple sclerosis, demyelinating polyneuropathy (CIDP) and Guillain-Barré syndrome (GBS), is thought to be due to the presence of autoimmune reaction against nerves, including myelin components (Ho et al., 1998; Kwa et al., 2003; Steck et al., 1998). In order, to mimic antibody-induced demyelination, an in vitro system was setup where organotypic hippocampal slice cultures were treated for two days by anti-MOG (myelin oligodendrocyte glycoprotein) antibody in combination with baby rabbit complement proteins. The treatment results in a specific demyelination since isotype matching control immunoglobulin treatment do not induce significant demyelination, apart from some cytotoxic activities of the baby rabbit serum per se. This system was used to test the potential promyelinatory effect of IL-17F. In this paradigm, IL-17F was added 48 hours after demyelinating treatment, and was given every other day for 8 additional days. At the end of the experiment, the MBP level was monitored by ELISA (see Example 4 for details).

### Materials and Methods

### Organotypic hippocampal slice cultures

Organotypic hippocampal slice cultures were prepared according to the method of Stoppini et al. (Stoppini et al., 1991). Briefly, hippocampi were obtained from five day-old C57/BI6 mice. Using a Mcillvain tissue chopper, 500-micron thick slices were cut. Slices were then disposed onto Millicell-CM inserts placed in 6 wells plates containing 1ml of culture medium (50%MEM, 25%HBSS, 25% horse serum). Cultures were maintained in 5% CO2 at 37°C during the 6^{th} days and then transferred at 33°C. Medium was changed every 3 days.

### Demyelinating protocol

Organotypic hippocampal slice cultures section, were treated at the end of developmental myelination that occurs after 21 days in vitro (DIV).

Demyelination was induced by treating slices with anti-MOG antibodies associated with baby rabbit complement proteins (1/60-1/30 depending of the batch; CL-3441, Cedarlane) during 2 days in 25% horse serum containing medium.

As controls, slices were treated with IgG1 irrelevant antibodies (60ug/ml; M-7894, Sigma) and complement, or slices were untreated.

At the end of the treatment, slices (6 per group) were lysed in triple detergent buffer and myelin level content analyzed by MBP ELISA (Example 4).

IL-17F (1 ng/ml, 10 ng/ml or 100 ng/ml) was given at the end of the demyelinating treatment. Medium was changed every other day for 8 additional days and IL-17F treatment was renewed at each change.

The results shown in Fig. 7 represent a pool of individual data of two independent experiments.

### Results

The results of this experiment are shown in Fig. 7. Adding 10ng/ml or 100 ng/ml of IL-17F to the medium after induction of demyelination significantly increased MBP content during the remyelination phase.

### Conclusion

In an autoimmune mediated demyelination model, IL-17F induces remyelination following a demyelination episode induced by anti-MOG and complement.

### EXAMPLE 7: IL-17F increases IL-6 release in mice DRG (Fig. 8)

### Introduction

Several evidences suggest that IL-6 is regulator of neuronal survival and differentiation in the central and peripheral nervous system. The present study was carried out to evaluate IL-6 production by DRG (dorsal root ganglion) explants treated with 3 members of the IL-17 family.

### Material and method

DRGs were isolated from P2-5 days old C57/black mice. DRG explants were plated in a 96 well plate coated with poly-D-lysine, in RPMI 1640 medium (Invitrogen, supplemented only with glutamine and pen/strep), two explants/well, and treated with IL-17A, IL-17F (at 0.1, 1, 10 or 100 ng/ml) or IL-17E (at 1, 10 or 100 ng/ml), all from Peprotech. The negative control consisted in RPMI medium alone, and NGF was used as a positive control (100 ng/ml), because of its well-described effects on neurite outgrowth. Explants were incubated at 5% CO2, 37 °C for 3 days. Supernatants were collected and tested for mouse IL-6, using a commercial ELISA (from R&D).

### Results

As shown in Fig. 8, IL-6 release in mouse DRG was significantly induced by IL-17A and IL-17F, in a concentration-dependent manner. In comparison, IL-17E, at similar concentrations induced little or no IL-6 release.

### Conclusion

Two members of the IL-17 family, IL-17A and IL-17F, were able to significantly induce IL-6 release by mouse DRG.

### EXAMPLE 8: Effect of IL-17F on MOG-induced experimental autoimmune encephalomyelitis (EAE) in mice (Fig. 9)

### Introduction

IL-17F is a cytokine with pleiotropic functions, including immuno-regulatory properties. IL-17F may also play a role as a modulator of neuronal and glial function. The purpose of this study was to test the therapeutic effect of IL-17F in the model of MOG-induced EAE in mice.

### Materials and methods

The method of induction of EAE used for this study has been adapted from the protocol published by Sahrbacher et al. (1998). All experiments were carried out in accordance with institutional guidelines.

### Animals

C57 BL/6JICO female mice from the IFFA CREDO (Saint Germain sur l'Arbresle, France) colony, supplied by Charles River Italia (Calco, Lecco, Italy), were used. This selected strain has documented susceptibility to EAE.
The animals were acclimated at least 5 days before the study was initiated. In this period the animals were observed daily to ascertain their fitness for the study.
*Age and body weight:* About 8-week old; 18-22 g.

*Housing*: animals were kept in the following conditions
- 10 animals/cage in air-conditioned rooms
- Temperature: 22°C ± 2
- relative humidity: 55% ± 10
- Air changes: about 15-20/hour filtered on HEPA 99.99%
- Light: 12 hour cycle (7 a.m. - 7 p.m.)

*Diet:* GLP 4RF25 top certificate pelleted diet produced by Charles River Italia's feed licensee Mucedola S.r.i., Settimo Milanese. To facilitate nourishment of sick animals, from day 7 wet pellets were placed every day on the cage bottom.

*Water:* From the municipal main watering system. Water was filtered and distributed "ad libitum" to the animals by an automatic valve system.

### Immunization procedure

3 groups of 10 female mice were immunized (day=0) by injecting s.c. in the left flank 0.2 ml of an emulsion composed of 200 µg MOG₃₅₋₅₅ peptide (Neosystem, Strasbourg, France) in Complete Freund's Adjuvant (CFA, Difco, Detroit, U.S.A.) containing 0.5 mg of *Mycobacterium tuberculosis.* Immediately after, they received an i.p. injection of 500 ng pertussis toxin (List Biological Lab., Campbell, CA, U.S.A.) dissolved in 400 µl of buffer (0.5 M NaCl, 0.017% Triton X-100, 0.015 M Tris, pH=7.5). On day 2 the animals were given a second i.p. injection of 500 ng pertussis toxin. On day 7, the mice received a second dose of 200 µg of MOG₃₅₋₅₅ peptide in CFA injected s.c. in the right flank. Starting approximately from day 8-10, this procedure results in a gradually progressing paralysis, arising from the tail and ascending up to the forelimbs.

### Treatment protocol

The treatment of the groups immunized as described above, was started for each animal at the appearance of a clinical score ≥1 and continued for 21 consecutive days. The animals were treated daily as follows:
***Group* 1**: control group treated by s.c. route with vehicle alone (0.9% NaCl + 0.1% BSA in a volume of 10 ml/kg).
***Group* 2**: mIFNβ was given by s.c. route at the dose of 20,000 U/mouse in a volume of 200 µl/mouse.
***Group 3***: treated with IL-17F at 2.5 µg/kg administered by s.c. route in a volume of 10 ml/kg.

Vehicle (0.9% NaCl + 0.1% BSA) was used to dilute IL-17F and PBS to dilute mIFNβ to the appropriate concentrations.

### Clinical observations

Starting from day 7 post-immunization the animals were individually examined for the presence of paralysis by means of a clinical score as follows:
- 0 = no sign of disease
- 0.5 = partial paralysis
- 1 = tail paralysis
- 1.5 = tail paralysis + partial unilateral hindlimb paralysis
- 2 = tail paralysis + hindlimb weakness or partial hindlimb paralysis
- 2.5 = tail paralysis + partial hindlimb paralysis (lowered pelvi)
- 3 = tail paralysis + complete hindlimb paralysis
- 3.5 = tail paralysis + complete hindlimb paralysis + incontinence
- 4 = tail paralysis + hindlimb paralysis + weakness or partial paralysis of forelimbs
- 5 = moribund or dead

Observation of the animals took place in a quiet room. Clinical signs were monitored daily in each group of treatment in a blind fashion by a technician who was unaware of treatments. Body weight of the animals was monitored daily.

Animals considered to be in pain distress or in moribund condition were examined by the staff veterinarian or authorized personnel and, if necessary, humanely sacrificed to minimize undue pain or suffering.

### Data evaluation

Results of clinical examinations were expressed as the mean (± SEM) score within each group. The effects of the test substances (IL-17F and mIFNβ) were compared with that of the vehicle-treated positive control group (group 1). Differences of clinical score values among groups was analysed by Kruskal-Wallis test followed, in case of significance, by the pairwise Wilcoxon test, at each measurement time. The S- Plus@ software was used.

### Results

Mice treated with vehicle developed the disease reaching a peak at day 4 after onset of the disease (clinical score ≥ 1) with a mean score value of 2.3 ± 0.2 (Fig 9). Chronic treatment with IL-17F at the dose of 2.5 µg/kg s.c. showed a slight but continuous effect on clinical score. The reference compound mIFNβ administered at the onset (clinical score ≥ 1) at the dose of 20,000 U/mouse s.c. significantly reduced the clinical disability as showed in Fig. 9.

### Conclusion

These data suggest that IL-17F given at relatively low dose has no pro-inflammatory effect, and rather improves clinical effects in the mouse EAE, and may therefore be a treatment for multiple sclerosis.

### EXAMPLE 9: Anti-inflammatory effect of IL-17F on spinal cord inflammation induced by sciatic nerve cut (Fig. 10)

### Introduction

The present study was carried out to evaluate the effect of IL-17F in a model of retrograde spinal cord inflammation following a sciatic nerve cut. In this model dexamethasone was used as positive control.

### Materials and Methods

### Animals

Twenty-one 2 week-old baby Wistar rats from two different litters (Charles River, France) were used. They were divided into 4 groups (n = 4 to 6): (a) vehicle nerve cut operated group; (c) nerve cut/IL-17F (0.1 mg/kg); (d) nerve cut/IL-17F (0.01 mg/kg); (e) dexamethasone (0.5 mg/kg)

The animals were kept with their respective mother in individual cages and maintained in a room with controlled temperature (21-22°C) and a reversed light-dark cycle (12h/12h) with food and water available *ad libitum*. All experiments were carried out in accordance with institutional guidelines.

### Lesion of the sciatic nerve

The animals were anaesthetized by inhalation of 3% Isofluran^{®} (Baxter). The right sciatic nerve was surgically exposed at mid thigh level and cut at 5 mm proximal to the trifurcation of the sciatic nerve. The lesion was closed with surgical glue.

### Planning of experiments and pharmacological treatment

The day of nerve cut surgery was considered as dpl 0 (dpl = day post lesion). Body weight and survival rate were recorded every day.

IL-17F or dexatmethasone were administered daily by subcutaneous injections (s.c.) route from the day of the surgery (4 hours before surgery). The treatments were repeated the following two days. Vehicle consisted of sodium acetate 50mM + NaCl 150mM pH5 and was diluted in NaCl 0.9% to get the same concentration than animals which received IL-17F. Animals were all sacrificed at 3 dpl with an overdose of pentobarbital (100 mg/kg), and perfused with PBS. Spinal cords were then removed and processed for immunostaining.

### Flow cytometry (FACS) analyses of the leukocyte infiltration

At sacrifice (3 day post lesion), the animals were perfused with PBS. The spinal cords were then dissected out (anterior part and posterior part), minced and incubated in collagenase (8 mg/ml) and 1% DNAse for 2h at 37C. The anterior part represents the part of the spinal cord that has no inflammation, whereas the posterior part represents the inflamed part (contains the L4-L5 lumbar region where the sciatic nerve has its roots). The pieces were then mechanically dissociated with a pipet and passed through a 70 micron mesh. The cells were then washed and blocked in 1 %BSA, 0.01 % NaN3 and rabbit IgGs (3 ug/ml) for 30 min, and incubated in phycoerthryin-conjugated mouse anti-CD45 and fluorescein-conjugated mouse anti-CD11 antibodies (25 ug/ml, from R&D) for 45 min. The cells were then analyzed on FACSCalibur from Becton Dickinson. The percent of CD45 and CD11 positive cells was reported on a graph, for the anterior and posterior parts of the spinal cords, for all treatments. Four to six animals were taken for each treatment.

### Data analysis

Global analysis of the data was performed using one-way ANOVA. Dunnett's test was used further, and data were compared to the "vehicle" control. The level of significance was set at a: p < 0.001; b: p < 0.01; c: p < 0.05; d: p < 0.1. The results were expressed as mean ± standard error of the mean (s.e.m.).

### Results

### Animal weight

Animals treated with dexamethasone lost significant body weight during the study (not shown). All animals survived the nerve cut procedures and throughout the study.

### Flow cytometry (FACS) analyses of the leukocyte infiltration (Fig. 10)

Three days after the nerve cut, the leukocyte infiltration in the posterior part of the spinal cord was reduced in the animals treated with IL-17F or dexamethasone in comparison to the vehicle-treated animals.

### Conclusions

Recombinant human IL-17F injected subcutaneously induced a decrease in the number of inflamed cells within the spinal cord, suggesting that IL-17F exerts anti-inflammatory effects.

### EXAMPLE 10: Effect of IL-17F on cytokine release measured in the LPS induced TNF-α release animal model (Fig. 11)

### Materials and methods

The model of lipopolysaccharide (LPS)-induced TNF-α release in mice was set up according to the patent application WO98/38179. LPS (O111:B4; SIGMA) was injected (0.3 mg/kg, i.p.) in C3H/HeN mice (Charles River, France). Ninety minutes later blood was sampled and plasma TNF-α was determined using an ELISA kit (R&D). Recombinant human IL-17F and dexamethasone were diluted in NaCl and injected 15 minutes prior to LPS administration.

Two experients were performed and the animals treated as follows :
1. Vehicle (NaCl), IL-17F at 1 mg/kg, 500, 100 µg/kg, administered by i.v. route or dexamethasone at 0.1 mg/kg administered by s.c. route.
2. Vehicle (NaCl) s.c. and i.v., IL-17Fat 500 µg/kg administered via s.c. or i.v. routes.

### Results

In the first experiment (Fig. 11.A), IL-17F was shown to inhibit LPS-induced TNF-α release in an inverted dose-dependent manner.

In the second experiment (Fig. 11.B) IL-17F administered by i.v. route inhibited LPS-induced TNF-α release by 52%. A decrease in the TNF-α release was also observed with subcutaneous administration of IL-17F, despite a slightly less efficient inhibition.

### EXAMPLE 11: Effect of Therapeutic administration of IL-17F on Diabetic Peripheral Neuropathy in the rat (Fig. 12)

Diabetic peripheral neuropathy represents a common complication of diabetes that can affect virtually every tissue of the body and cause significant morbidity and mortality. It occurs in more than 50% of patients who have been hyperglycemic for more than 15 years and involves degeneration of myelinated fibers and defects of sensitivity, that may become incapacitating and irreversible. Patients with sensorimotor neuropathy may suffer from causalgia, tactile hypersensitivity and paraesthesia. At a later stage, disturbances of motor function can appear. This disease is a polyneuropathy that affects various fiber classes, leads to reduction of nerve conduction velocity, axonal degeneration, and neuronal loss.

In this example diabetic neuropathy was induced by one acute administration of streptozotocin (=STZ - 55 mg/kg, I.V.), an antibiotic isolated from Streptomyces achromogenes fermentation broth, which is known to induce diabetes in several animal species. In this model, demyelination and axonal loss occur.

In this the IL-17F effect was compared to the effect of subcutaneous administred IL-6, which has already been demonstrated to display neuroprotective activity.

### Materials and methods

### Disease Induction

Sprague-Dawley male rats (200-225 g) were rendered diabetic by intravenous injection of streptozotocin (55 mg/kg). One week later, diabetes was confirmed in STZ-injected rats by obtaining a blood sample from the tail vein and by measuring the glucose concentration using a glucose monitor. Only STZ-treated rats with plasma-glucose > 260 mg/dl were deemed "diabetic".
60 animals were randomly assigned to the following groups (12 rats per group):

| **Group (n = 12)** | **Test Substance** | **Dose** | **Administration route** | **Frequency** | **Treatment period (days post immunization)** |
|---|---|---|---|---|---|
| Gr. 1 | Non-diabetic | - | s.c. | Daily | 11 to 40 |
| Gr.2 | Vehicle | - | s.c. | Daily | 11 to 40 |
| Gr.3 | IL-17F | 0.3 mg/kg | s.c. | Daily | 11 to 40 |
| Gr.4 | IL-17F | 0.1 mg/kg | s.c. | Daily | 11 to 40 |
| Gr.5 | IL-17F | 0.03 mg/kg | s.c. | Daily | 11 to 40 |
| Gr.6 | r-hIL-6 | 30 µg/kg | s.c. | Daily | 11 to 40 |

### Von Frey filament test (for allodynia)

Rats were placed on a metallic grid floor. The nociceptive testing was done by inserting the von Frey filament (Bioseb, France) through the grid floor and applying it to the plantar surface of the hind paw. A trial consisted of several applications of the different von Frey filaments (at a frequency of 1-1.5 s). The von Frey filaments were applied from filament 10 g to 100 g. The pressure that produces a brisk withdrawal of hind paw was considered as threshold value. Cutoff value was set to 100 g.

### Electrophysiological measurements (at day 40 post-STZ administration)

Electrophysiological recordings were performed using a Neuromatic 2000M electromyograph (EMG) (Dantec, Les Ulis, France). Rats were anaesthetized by intraperitoneal injection of 60 mg/kg ketamine chlorhydrate (Imalgene 500®, Rhône Mérieux, Lyon, France). The normal body temperature was maintained at 30°C with a heating lamp and controlled by a contact thermometer (Quick, Bioblock Scientific, IIIkirch, France) placed on the tail surface.

**CMAP** (compound muscle action potential) was recorded in the gastrocnemius muscle after stimulation of the sciatic nerve. A reference electrode and an active needle were placed in the hindpaw. A ground needle was inserted on the lower back of the rat. Sciatic nerve was stimulated with a single 0.2 ms pulse at a supramaximal intensity. The velocity of the motor wave was recorded.

**SNCV** (Sensitive nerve conduction velocity) was also recorded. The tail skin electrodes were placed as follows: a reference needle inserted at the base of the tail and an anode needle placed 30 mm away from the reference needle towards the extremity of the tail. A ground needle electrode was inserted between the anode and reference needles. The caudal nerve was stimulated with a series of 20 pulses (for 0.2 ms) at an intensity of 12.8 mA. The velocity was expressed as m/s.

### Protocol

- Day -7: baseline (EMG)
- Day 0: induction by the streptozotocin
- Day 10: glycemia control
- Day 11: Onset of the treatment
- Day 25: pain assessment
- Day 40: EMG and pain assessment

### Data analysis

For behavioral and EMG studies a global analysis of data was made using one factor or repeated measures analysis of variance (ANOVA). The Fisher test or the Student-Newman-Keuls T test were used to make multiple comparisons of individual test groups with control group or between paired groups.

### Results

### Von Frey filament test (for allodynia) (Fig 12.A):

STZ-induced diabetes resulted in significant allodynia at D25 and D40. This effect was reversed by IL-6 treatment at D25 but was lost by D40.

Although not reaching statistical significance, IL-17F treatment at 0.1 and 0.3 mg/kg partially reversed allodynia at D25 (p=0.069, p=0.085, respectively) suggesting that IL-17F may have an effect on fiber sensitivity.

### Electrophysiological measurements (at day 40 post-STZ administration)

### CMAP (indicative of motor nerve fiber conduction), Fig 12.B:

STZ-induced diabetes delayed significantly the compound motor action potentials in comparison to control/vehicle animals revealing potential ongoing motor nerve damages. This effect was significantly reversed by IL6 treatment and IL-17F at the dose of 0.3mg/kg suggesting a beneficial effect of IL-17F on motor nerve functionality.

### SNCV (Sensitive nerve conduction velocity), Fig 12.C:

STZ-induced diabetes reduced significantly sensory nerve velocity. IL6 treatment and IL-17F at 0.3 and 0.1 mg/kg significantly improved SNCV suggesting that IL-17F may have also a beneficial effect on sensory fibers. Differences observed between control/vehicle groups at D-7 and D40 reflects the naturally occurring increase in SNCV due to maturation of the nervous system (Biessels et al., 1999)

### Conclusions

IL-17F treatment in an STZ-induced diabetes model revealed a potential beneficial effect in a behavioral read-out of mechanical allodynia that was paralleled by significant improvement in motor and sensory electrophysiological assessment.

### REFERENCES

1. Abramsky,O. and Ovadia,H. (1997). Frontiers in Multiple Sclerosis: Clinical Research and Therapy. Martin Dunitz Ltd).
2. Aggarwal,S., Ghilardi,N., Xie,M.H., de Sauvage,F.J., and Gumey,A.L. (2003). Interleukin-23 promotes a distinct CD4 T cell activation state characterized by the production of interleukin-17. J Biol Chem 278, 1910-1914.
3. Aggarwal,S. and Gumey,A.L. (2002). IL-17: prototype member of an emerging cytokine family. J Leukoc Biol 71, 1-8.
4. Altschul,S.F., Gish,W., Miller,W., Myers,E.W., and Lipman,D.J. (1990). Basic local alignment search tool. J. Mol. Biol. 215, 403-410.
5. Altschul,S.F., Madden,T.L., Schaffer,A.A., Zhang,J., Zhang,Z., Miller,W., and Lipman,D.J. (1997). Gapped BLAST and PSI-BLAST: a new generation of protein database search programs. Nucleic Acids Res. 25, 3389-3402.
6. Anonymous (1998). Randomised double-blind placebo-controlled study of interferon beta-1a in relapsing/remitting multiple sclerosis. PRISMS (Prevention of Relapses and Disability by Interferon beta-1a Subcutaneously in Multiple Sclerosis) Study Group . Lancet 352, 1498-1504.
7. Barres,B.A. and Raff,M.C. (1999). Axonal control of oligodendrocyte development. J Cell Biol 147, 1123-1128.
8. Biessels,G.J., Cristino,N.A., Rutten,G.J., Hamers,F.P., Erkelens,D.W., and Gispen,W.H. (1999). Neurophysiological changes in the central and peripheral nervous system of streptozotocin-diabetic rats. Course of development and effects of insulin treatment. Brain 122 ( Pt 4), 757-768.
9. Capello.E., Voskuhl.R.R., McFarland,H.F., and Raine,C.S. (1997). Multiple sclerosis: reexpression of a developmental gene in chronic lesions correlates with remyelination. Ann. Neurol. 41, 797-805.
10. Derynck,R., Content,J., DeClercq,E., Volckaert,G., Tavemier,J., Devos,R., and Fiers,W. (1980). Isolation and structure of a human fibroblast interferon gene. Nature 285, 542-547.
11. Devereux,J., Haeberli,P., and Smithies,O. (1984). A comprehensive set of sequence analysis programs for the VAX. Nucleic Acids Res. 12, 387-395.
12. Dubois-Dalcq,M. and Murray,K. (2000). Why are growth factors important in oligodendrocyte physiology? Pathol. Biol (Paris) 48, 80-86.
13. Femandez,P.A., Tang,D.G., Cheng,L., Prochiantz,A., Mudge,A.W., and Raff,M.C. (2000). Evidence that axon-derived neuregulin promotes oligodendrocyte survival in the developing rat optic nerve. Neuron 28, 81-90.
14. Fonnum,F. (1969). Radiochemical micro assays for the determination of choline acetyltransferase and acetylcholinesterase activities. Biochem J 115, 465-472.
15. Giannakopoulos,P., Kovari,E., French,L.E., Viard,I., Hof,P.R., and Bouras,C. (1998). Possible neuroprotective role of clusterin in Alzheimer's disease: a quantitative immunocytochemical study. Acta Neuropathol. (Berl) 95, 387-394.
16. Grantham,R. (1974). Amino acid difference formula to help explain protein evolution. Science 185, 862-864.
17. Hajihosseini,M., Tham,T.N., and Dubois-Dalcq,M. (1996). Origin of oligodendrocytes within the human spinal cord. J Neurosci 16, 7981-7994.
18. Hartung,H.P., van der Meche,F.G., and Pollard,J.D. (1998). Guillain-Barre syndrome, CIDP and other chronic immune-mediated neuropathies. Curr Opin Neurol. 11, 497-513.
19. Ho,T.W., McKhann,G.M., and Griffin,J.W. (1998). Human autoimmune neuropathies. Annu Rev Neurosci. 21, 187-226.
20. Hurst,S.D., Muchamuel,T., Gorman,D.M., Gilbert,J.M., Clifford,T., Kwan,S., Menon,S., Seymour,B., Jackson,C., Kung,T.T., Brieland,J.K., Zurawski,S.M., Chapman,R.W., Zurawski,G., and Coffman,R.L. (2002). New IL-17 family members promote Th1 or Th2 responses in the lung: in vivo function of the novel cytokine IL-25. J Immunol 169, 443-453.
21. Hymowitz,S.G., Filvaroff,E.H., Yin,J.P., Lee,J., Cai,L., Risser,P., Maruoka,M., Mao,W., Foster,J., Kelley,R.F., Pan,G., Gumey,A.L., de Vos,A.M., and Starovasnik,M.A. (2001). IL-17s adopt a cystine knot fold: structure and activity of a novel cytokine, IL-17F, and implications for receptor binding. EMBO J 20, 5332-5341.
22. Jones,S.E. and Jomary,C. (2002). Clusterin. Int. J. Biochem. Cell Biol. 34, 427-431.
23. Kawaguchi,M., Kokubu,F., Matsukura,S., leki,K., Odaka,M., Watanabe,S. , Suzuki,S., Adachi,M., and Huang,S.K. (2003). Induction of C-X-C chemokines, growth-related oncogene alpha expression, and epithelial cell-derived neutrophil-activating protein-78 by ML-1 (interleukin-17F) involves activation of Raf1-mitogen-activated protein kinase kinase-extracellular signal-regulated kinase 1/2 pathway. J Pharmacol Exp Ther. 307, 1213-1220.
24. Kawaguchi,M., Onuchic,L.F., and Huang,S.K. (2002). Activation of extracellular signal-regulated kinase (ERK)1/2, but not p38 and c-Jun N-terminal kinase, is involved in signaling of a novel cytokine, ML-1. J Biol Chem 277, 15229-15232.
25. Kawaguchi,M., Onuchic,L.F., Li,X.D., Essayan,D.M., Schroeder,J., Xiao,H.Q., Liu,M.C., Krishnaswamy,G. , Germino,G., and Huang,S.K. (2001). Identification of a novel cytokine, ML-1, and its expression in subjects with asthma. J Immunol 167, 4430-4435.
26. Kuhn,G., Lie,A., Wilms,S., and Muller,H.W. (1993). Coexpression of PMP22 gene with MBP and P0 during de novo myelination and nerve repair. Glia 8, 256-264.
27. Kwa,M.S., van Schaik,I.N., De Jonge,R.R., Brand,A., Kalaydjieva,L., van Belzen,N., Vermeulen,M., and Baas,F. (2003). Autoimmunoreactivity to Schwann cells in patients with inflammatory neuropathies. Brain 126, 361-375.
28. Marchionni,M.A., Cannella.B., Hoban,C., Gao,Y.L., Garcia-Arenas,R., Lawson,D., Happel,E., Noel,F., Tofilon,P., Gwynne,D., and Raine,C.S. (1999). Neuregulin in neuron/glial interactions in the central nervous system. GGF2 diminishes autoimmune demyelination, promotes oligodendrocyte progenitor expansion, and enhances remyelination. Adv. Exp Med Biol 468, 283-295.
29. Mark,D.F., Lu,S.D., Creasey,A.A., Yamamoto,R., and Lin,L.S. (1984). Site-specific mutagenesis of the human fibroblast interferon gene. Proc. Natl. Acad. Sci. U. S. A 81, 5662-5666.
30. McDonald,J.W., Althomsons,S.P., Hyrc,K.L., Choi,D.W., and Goldberg,M.P. (1998). Oligodendrocytes from forebrain are highly vulnerable to AMPA/kainate receptor-mediated excitotoxicity. Nat Med 4, 291-297.
31. McMahon,S.B. and Priestley,J.V. (1995). Peripheral neuropathies and neurotrophic factors: animal models and clinical perspectives. Curr. Opin. Neurobiol. 5, 616-624.
32. Messmer-Joudrier,S., Sagot,Y., Mattenberger,L., James,R.W., and Kato,A.C. (1996). Injury-induced synthesis and release of apolipoprotein E and clusterin from rat neural cells. Eur J Neurosci 8, 2652-2661.
33. Moseley,T.A., Haudenschild,D.R., Rose,L., and Reddi,A.H. (2003). Interleukin-17 family and IL-17 receptors. Cytokine Growth Factor Rev 14, 155-174.
34. Pantoni,L., Garcia,J.H., and Gutierrez,J.A. (1996). Cerebral white matter is highly vulnerable to ischemia. Stroke 27, 1641-1646.
35. Pearson,W.R. (1990). Rapid and sensitive sequence comparison with FASTP and FASTA. Methods Enzymol. 183, 63-98.
36. Pearson,W.R. and Lipman,D.J. (1988). Improved tools for biological sequence comparison. Proc. Natl. Acad. Sci. U. S. A 85, 2444-2448.
37. Prineas,J.W., Bamard,R.O., Kwon,E.E., Sharer,L.R., and Cho,E.S. (1993). Multiple sclerosis: remyelination of nascent lesions. Ann. Neurol. 33, 137-151.
38. Sahrbacher,U.C., Lechner,F., Eugster,H.P., Frei,K. , Lassmann,H., and Fontana,A. (1998). Mice with an inactivation of the inducible nitric oxide synthase gene are susceptible to experimental autoimmune encephalomyelitis. Eur J Immunol 28, 1332-1338.
39. Scherer,S.S. (1997). Molecular genetics of demyelination: new wrinkles on an old membrane. Neuron 18, 13-16.
40. Scolding,N. and Lassmann,H. (1996). Demyelination and remyelination. Trends Neurosci 19, 1-2.
41. Shepard,H.M., Leung,D., Stebbing,N., and Goeddel,D.V. (1981). A single amino acid change in IFN-beta1 abolishes its antiviral activity. Nature 294, 563-565.
42. Smith,T.F. and Waterman,M.S. (1981). Identification of common molecular subsequences. J. Mol. Biol. 147, 195-197.
43. Stames,T., Robertson,M.J., Sledge,G., Kelich,S., Nakshatri,H., Broxmeyer,H.E., and Hromas,R. (2001). Cutting edge: IL-17F, a novel cytokine selectively expressed in activated T cells and monocytes, regulates angiogenesis and endothelial cell cytokine production. J Immunol 167, 4137-4140.
44. Steck,A.J., Schaeren-Wiemers,N., and Hartung,H.P. (1998). Demyelinating inflammatory neuropathies, including Guillain-Barre syndrome. Curr Opin Neurol. 11, 311-318.
45. Stoppini,L., Buchs,P.A., and Muller,D. (1991). A simple method for organotypic cultures of nervous tissue. J Neurosci. Methods 37, 173-182.
46. Trougakos,I.P. and Gonos,E.S. (2002). Clusterin/apolipoprotein J in human aging and cancer. Int. J. Biochem. Cell Biol. 34, 1430-1448.

## Claims

1. Use of IL-17F or of a nucleic acid molecule encoding IL-17F, for the manufacture of a medicament for the treatment and/or prevention of a neurologic disease wherein said nucleic acid molecule comprises a nucleic acid sequence of SEQ ID NO: 2 or a nucleic acid sequence encoding a polypeptide comprising an amino acid sequence selected from the group consisting of:
(a) a polypeptide comprising SEQ ID NO: 1;
(b) a polypeptide comprising amino acids 11 to 163 of SEQ ID NO: 1;
(c) a polypeptide comprising amino acids 21 to 163 of SEQ ID NO: 1;
(d) a polypeptide comprising amino acids 31 to 163 of SEQ ID NO: 1;
(e) a polypeptide comprising amino acids 55 to 163 of SEQ ID NO: 1;

2. The use according to claim 1, wherein the neurologic disease is associated with inflammation.

3. The use according to claim 2, wherein the inflammation is neuro-inflammation.

4. The use according to any of the preceding claims, wherein the neurologic disease is selected from the group consisting of traumatic nerve injury, stroke, demyelinating diseases of the CNS or PNS, neuropathies and neurodegenerative diseases.

5. The use according to any of the preceding claims, wherein the neurologic disease is caused by a congenital metabolic disorder.

6. The use according to any of the preceding claims, wherein the neurologic disease is a peripheral neuropathy.

7. The use according to claim 6, wherein the neurologic disease is diabetic neuropathy.

8. The use according to claim 4, wherein the demyelinating disease is multiple sclerosis (MS).

9. The use according to claim 8, wherein multiple sclerosis is relapsing-remitting multiple sclerosis.

10. The use according to claim 4, wherin the demyelinating disease is selected from chronic inflammatory multiple sclerosis, demyelinating polyneuropathy (CIDP) and Guillain-Barré syndrome (GBS),

11. The use according to claim 4, wherein the neurodegenerative disease is selected from Alzheimer's disease, Parkinson's disease, Huntington's disease and amyotrophic lateral sclerosis (ALS).

12. The use according to any of the preceding claims, wherein IL-17F is selected from the group consisting of:
(a) a polypeptide comprising SEQ ID NO: 1;
(b) a polypeptide comprising amino acids 11 to 163 of SEQ ID NO: 1;
(c) a polypeptide comprising amino acids 21 to 163 of SEQ ID NO: 1;
(d) a polypeptide comprising amino acids 31 to 163 of SEQ ID NO: 1;
(e) a polypeptide comprising amino acids 55 to 163 of SEQ ID NO: 1;
(f) a salt or a fused protein of any of (a) to (e).

13. The use according to any preceding claims, wherein IL-17F is dimeric.

14. The use according to any of the preceding claims, wherein IL-17F is fused to a carrier molecule, a peptide or a protein that promotes the crossing of the blood brain barrier.

15. The use according to any of the preceding claims, wherein the IL-17F is PEGylated.

16. The use according to claim 12, wherein the fused protein comprises an immunoglobulin (Ig) fusion.

17. The use according to any of the preceding claims, wherein the medicament further comprises an interferon and/or osteopontin and/or clusterin, for simultaneous, sequential, or separate use.

18. The use according to claim 17, wherein the interferon is interferon-β.

19. The use according to any of the preceding claims, wherein the IL-17F is used in an amount of about 0.001 to 100 mg/kg of body weight, or about 0.01 to 10 mg/kg of body weight or about 9, 8, 7, 6, 5, 4, 3, 2 or 1 mg/kg of body weight or about 0.1 to1 mg/kg of body weight.

20. Use according to any preceding claims, wherein the nucleic acid molecule further comprises an expression vector sequence.

## Patentansprüche

1. Verwendung von IL-17F oder eines Nukleinsäuremoleküls, das IL-17F kodiert, zur Herstellung eines Medikaments zur Behandlung und/oder Prävention einer neurologischen Erkrankung, wobei das Nukleinsäuremolekül eine Nukleinsäursequenz der SEQ ID NO: 2 umfasst oder eine Nukleinsäuresequenz, die ein Polypeptid kodiert, das eine Aminosäuresequenz umfasst, die ausgewählt ist aus der Gruppe bestehend aus:
(a) einem Polypeptid, das die SEQ ID NO: 1 umfasst;
(b) einem Polypeptid, das die Aminosäuren 11 bis 163 der SEQ ID NO: 1 umfasst;
(c) einem Polypeptid, das die Aminosäuren 21 bis 163 der SEQ ID NO: 1 umfasst;
(d) einem Polypeptid, das die Aminosäuren 31 bis 163 der SEQ ID NO: 1 umfasst;
(e) einem Polypeptid, das die Aminosäuren 55 bis 163 der SEQ ID NO: 1 umfasst;

2. Verwendung nach Anspruch 1, wobei die neurologische Erkrankung mit Entzündung verbunden ist.

3. Verwendung nach Anspruch 2, wobei die Entzündung eine Nervenentzündung ist.

4. Verwendung nach einem der vorhergehenden Ansprüche, wobei die neurologische Erkrankung aus der Gruppe ausgewählt ist, die aus traumatischer Nervenverletzung, Schlaganfall, Entmyelinisierungskrankheiten des ZNS oder PNS, Neuropathien und neurodegenerativen Erkrankungen besteht.

5. Verwendung nach einem der vorhergehenden Ansprüche, wobei die neurologische Erkrankung durch eine angeborene Stoffwechselkrankheit verursacht ist.

6. Verwendung nach einem der vorhergehenden Ansprüche, wobei die neurologische Erkrankung eine periphere Neuropathie ist.

7. Verwendung nach Anspruch 6, wobei die neurologische Erkrankung diabetische Neuropathie ist.

8. Verwendung nach Anspruch 4, wobei die Entmyelinisierungskrankheit Multiple Sklerose (MS) ist.

9. Verwendung nach Anspruch 8, wobei Multiple Sklerose rezidivierendremittierende Multiple Sklerose ist.

10. Verwendung nach Anspruch 4, wobei die Entmyelinisierungskrankheit ausgewählt ist aus chronisch-entzündlicher Multipler Sklerose, Entmyelinisierungs-Polyneuropathie (CIDP) und Guillain-Barré-Syndrom (GBS).

11. Verwendung nach Anspruch 4, wobei die neurodegenerative Erkrankung ausgewählt ist aus der Alzheimer-Krankheit, der Parkinson-Krankheit, Chorea Huntington und Amyotropher Lateralsklerose (ALS).

12. Verwendung nach einem der vorhergehenden Ansprüche, wobei IL-17F ausgewählt ist aus der Gruppe bestehend aus:
(a) einem Polypeptid, das die SEQ ID NO: 1 umfasst;
(b) einem Polypeptid, das die Aminosäuren 11 bis 163 der SEQ ID NO: 1 umfasst;
(c) einem Polypeptid, das die Aminosäuren 21 bis 163 der SEQ ID NO: 1 umfasst;
(d) einem Polypeptid, das die Aminosäuren 31 bis 163 der SEQ ID NO: 1 umfasst;
(e) einem Polypeptid, das die Aminosäuren 55 bis 163 der SEQ ID NO: 1 umfasst;
(f) einem Salz oder Fusionsprotein von einem von (a) bis (e).

13. Verwendung nach einem der vorhergehenden Ansprüche, wobei IL-17F dimer ist.

14. Verwendung nach einem der vorhergehenden Ansprüche, wobei IL-17F mit einem Trägermolekül, einem Peptid oder einem Protein, das die Überwindung der Blut-Hirn-Schranke fördert, fusioniert ist.

15. Verwendung nach einem der vorhergehenden Ansprüche, wobei das IL-17F PEGyliert ist.

16. Verwendung nach Anspruch 12, wobei das Fusionsprotein eine Immunglogulin- (Ig) Fusion umfasst.

17. Verwendung nach einem der vorhergehenden Ansprüche, wobei das Medikament weiterhin ein Interferon und/oder Osteopontin und/oder Clusterin zur gleichzeitigen, aufeinanderfolgenden oder separaten Verwendung umfasst.

18. Verwendung nach Anspruch 17, wobei Interferon Interferon-β ist.

19. Verwendung nach einem der vorhergehenden Ansprüche, wobei das IL-17F in einer Menge von etwa 0,001 bis 100 mg/kg Körpergewicht oder etwa 0,01 bis 10 mg/kg Körpergewicht oder etwa 9, 8, 7, 6, 5, 4, 3, 2 oder 1 mg/kg Körpergewicht oder etwa 0,1 bis 1 mg/kg Körpergewicht verwendet wird.

20. Verwendung nach einem der vorhergehenden Ansprüche, wobei das Nukleinsäuremolekül weiterhin eine Expressionsvektor-Sequenz umfasst.

## Revendications

1. Utilisation de l'interleukine 17F, ou d'une molécule d'acide nucléique codant l'interleukine 17F, en vue de la fabrication d'un médicament conçu pour le traitement et/ou la prévention d'une maladie neurologique, laquelle molécule d'acide nucléique comprend une séquence d'acide nucléique de la Séquence N° 2 ou une séquence d'acide nucléique qui code un polypeptide comprenant une séquence d'acides aminés, choisi dans l'ensemble constitué par les suivants :
a) un polypeptide comprenant la Séquence N° 1 ;
b) un polypeptide comprenant les résidus d'acides aminés n° 11 à 163 de la Séquence N° 1 ;
c) un polypeptide comprenant les résidus d'acides aminés n° 21 à 163 de la Séquence N° 1 ;
d) un polypeptide comprenant les résidus d'acides aminés n° 31 à 163 de la Séquence N° 1 ;
e) un polypeptide comprenant les résidus d'acides aminés n° 55 à 163 de la Séquence N° 1.

2. Utilisation conforme à la revendication 1, la maladie neurologique étant associée à une inflammation.

3. Utilisation conforme à la revendication 2, l'inflammation étant une neuro-inflammation.

4. Utilisation conforme à l'une des revendications précédentes, la maladie neurologique étant choisie dans l'ensemble constitué par les suivantes : lésion traumatique d'un nerf, attaque, maladies démyélinisantes du système nerveux central ou du système nerveux périphérique, neuropathies et maladies neurodégénératives.

5. Utilisation conforme à l'une des revendications précédentes, la maladie neurologique étant causée par un trouble métabolique congénital.

6. Utilisation conforme à l'une des revendications précédentes, la maladie neurologique étant une neuropathie périphérique.

7. Utilisation conforme à la revendication 6, la maladie neurologique étant une neuropathie diabétique.

8. Utilisation conforme à la revendication 4, la maladie démyélinisante étant une sclérose en plaques (SEP).

9. Utilisation conforme à la revendication 8, la sclérose en plaques étant une sclérose en plaques rémittente-récurrente.

10. Utilisation conforme à la revendication 4, la maladie démyélinisante étant choisie parmi la sclérose en plaques inflammatoire chronique, la polyneuropathie démyélinisante inflammatoire chronique (PDIC) et le syndrome de Guillain-Barré (SGB).

11. Utilisation conforme à la revendication 4, la maladie neurodégénérative étant choisie parmi la maladie d'Alzheimer, la maladie de Parkinson, la chorée de Huntington et la sclérose latérale amyotrophique (SLA).

12. Utilisation conforme à l'une des revendications précédentes, pour laquelle l'interleukine 17F est choisie dans l'ensemble constitué par les corps suivants :
a) un polypeptide comprenant la Séquence N° 1 ;
b) un polypeptide comprenant les résidus d'acides aminés n° 11 à 163 de la Séquence N° 1 ;
c) un polypeptide comprenant les résidus d'acides aminés n° 21 à 163 de la Séquence N° 1 ;
d) un polypeptide comprenant les résidus d'acides aminés n° 31 à 163 de la Séquence N° 1 ;
e) un polypeptide comprenant les résidus d'acides aminés n° 55 à 163 de la Séquence N° 1 ;
f) un sel ou une protéine de fusion de l'un des corps (a) à (e).

13. Utilisation conforme à l'une des revendications précédentes, pour laquelle l'interleukine 17F est sous forme dimère.

14. Utilisation conforme à l'une des revendications précédentes, pour laquelle l'interleukine 17F est fusionnée à une molécule vectrice, un peptide ou une protéine qui favorise la traversée de la barrière hématoencéphalique.

15. Utilisation conforme à l'une des revendications précédentes, pour laquelle l'interleukine 17F est pégylée.

16. Utilisation conforme à la revendication 12, pour laquelle la protéine de fusion comprend une immunoglobuline (Ig) fusionnée.

17. Utilisation conforme à l'une des revendications précédentes, le médicament devant en outre comprendre un interféron et/ou de l'ostéopontine et/ou de la clustérine, à utiliser simultanément, successivement ou séparément.

18. Utilisation conforme à la revendication 17, pour laquelle l'interféron est de l'interféron β.

19. Utilisation conforme à l'une des revendications précédentes, dans laquelle l'interleukine 17F est employée en une quantité d'environ 0,001 à 100 mg par kg de poids corporel, ou d'environ 0,01 à 10 mg par kg de poids corporel, ou d'environ 9, 8, 7, 6, 5, 4, 3, 2 ou 1 mg par kg de poids corporel, ou d'environ 0,1 à 1 mg par kg de poids corporel.

20. Utilisation conforme à l'une des revendications précédentes, pour laquelle la molécule d'acide nucléique comporte en outre une séquence de vecteur d'expression.
